# EUROPEAN PATENT APPLICATION

(11) **EP 4 696 702 A1**
(43) Date of publication of application: **18.02.2026**
(21) Application number: 24789081.7
(22) Date of filing: 12.04.2024
(51) Int. Cl.: C07K 14/005, C12N 9/00, C12N 15/70, A61K 39/125, A61P 31/14

(54) **RECOMBINANT EXPRESSION VECTOR FOR PRODUCTION OF VIRUS-LIKE PARTICLE-BASED MULTIVALENT NOROVIRUS VACCINE AND MANUFACTURING METHOD THEREFOR**

(30) Priority: 14.04.2023 KR 20230049610
(71) Applicant: Inthera Inc., Seoul 05836 (KR)
(72) Inventor: CHOI, Deog Young, Seoul 06673 (KR); JUNG, Hyun Gyo, Seoul 04726 (KR)
(74) Representative: Fuchs Patentanwälte Partnerschaft mbB
(86) International application number: PCT/KR2024/004954
(87) International publication number: WO 2024/215140

(57) **Abstract**

The present invention provides a fusion protein for promoting soluble expression of a norovirus antigen, and an expression vector thereof. More specifically, the present invention provides combinations of various genotypes of norovirus antigen proteins and RID mutants, in which the most efficient folding occurs when a norovirus antigen is used as a target protein to form a recombinant fusion protein, the yield of soluble expression of a fusion protein produced is improved, and the assembly efficacy and homogeneity of norovirus VLPs can be enhanced. Also, the present invention provides a method for rapidly mass-producing various other genotypes of norovirus VLPs, as well as GII.4, in E. coli by using mutant RID, thus enabling the development of a VLP-based multivalent vaccine comprising more genotypes.

## Description

### [Technical Field]

The present invention relates to a recombinant expression vector for producing a virus-like particle-based norovirus multivalent vaccine in *Escherichia coli* with high efficiency and rapid production and a preparation method thereof, and more specifically to a recombinant expression vector which is capable of soluble expression of various genotypes of antigen proteins of norovirus in *E. coli* with high efficiency and an efficient method for producing a norovirus multivalent vaccine by utilizing the same.

The present invention was carried out with the support of the research project of "Development of a VLP-type multivalent prophylactic vaccine for norovirus enteritis" (Project Unique Number: 1465035890, Research Period: October 6, 2020 to December 31, 2023) of the Infectious Disease Prevention and Treatment Technology Development Project supported by the Korean Ministry of Health and Welfare, and with the support of the research project of "Development of a formulation to enhance the long-term stability of a norovirus VLP-type vaccine candidate" (Project Unique Number: 2023112201, Research Period: July 1, 2021 to December 31, 2023) of the Regional Vaccine R&BD Infrastructure Activation Project supported by the Korean Ministry of Education.

### [Background Art]

Norovirus, which is the virus to which the technique of the present invention is applied, is one of the most important pathogens causing acute gastroenteritis and food poisoning. According to the announcement of the Foodborne Disease Burden Epidemiology Reference Group (FERG) under the WHO, approximately 700 million cases of norovirus infection are reported worldwide every year, with more than 200 million infections in children under the age of 5 and 50,000 deaths each year. Norovirus infection is mainly transmitted through person-to-person contact, aerosol and the fecal-oral route of patients, and it can also be transmitted through contaminated water or food. Norovirus can infect people of all ages, but it occurs more frequently in young infants and the elderly, and disease can occur with a very small amount of infection, the virus is excreted for a long period of time, and it is stable in heat at temperatures below 60°C. In addition, due to factors such as continuous antigen mutation and genetic recombination, it is impossible to acquire long-term immune antibodies, and a wide-ranging outbreak is possible, resulting in group infection cases in schools, hospitals, nursing homes and the like. The prominent symptoms of infection by the pathogen are abdominal pain, low-grade fever, vomiting and diarrhea. The general incubation period is 24 to 48 hours, and symptoms vary from asymptomatic to fever, vomiting, diarrhea, abdominal pain and dehydration, and in the case of the elderly in particular, severe dehydration can lead to death.

Norovirus is a non-enveloped virus belonging to the *Caliciviridae* family and is approximately 30 to 40 nm in diameter. It consists of a single-stranded (+)RNA of 7.6 kbp in length, and among the three open reading frames (ORF), ORF2 encodes the main structural protein VP1 that forms the shape of norovirus, and ORF3 encodes the VP2 protein, but it is not directly involved in structural formation. VP1 is approximately 59 kDa in total size and forms a dimer, and 90 dimers self-assemble, thereby forming a total of 180 VP1s to form one virus particle. The VP1 protein is composed of two domains, the S domain plays a role in forming the structure, and the P domain is known to be involved in inducing the actual immune response. As the P2 region of the P domain is known to bind to histo-blood group antigens (HBGAs), it has emerged as a target for vaccines.

Norovirus is classified into seven genogroups based on the entire amino acid sequence of VP1, with GI, GII and GIV causing human infection, and the most commonly detected genotype in outbreaks is GII.4, accounting for 50 to 70% of the overall prevalence. GII.4 changes antigenicity by mutation of the HBGA binding site of the P2 domain of VP1, and can evade the immune system through various genetic recombination, and the new global epidemic of a mutant type appears every 2 to 3 years. In addition to GII.4, there was a rapid outbreak of GII.17 in Asia from 2014 to 2015 (Chan et al., Nature Communications 2015), and GII.3 has been frequently detected since 2016 (Kuang, et al. Gut Pathog. 2019). The prevalence of GII.3 is increasing worldwide, and the GII.3/GII.2 genotype has been detected in Korea for the past three years. Norovirus is genetically very diverse, and new variants continue to emerge, and thus, it cannot be ruled out that it may become the dominant genotype replacing GII.4 in the future. Therefore, there is a strong need for the development of a multivalent vaccine that has preventive efficacy against as many genotypes as possible, including additional genotypes of the GII Genogroup, which account for more than 90% of the prevalence, for an effective norovirus infection prophylactic vaccine.

Traditionally, vaccines for viral infectious diseases have been manufactured in the form of live vaccines that attenuate the virus itself or inactivated killed vaccines that are cultured and purified. However, in the case of norovirus, since there is no known culture method for proliferating the virus, it is difficult to develop vaccine formulations of live and killed vaccines, and there is no suitable animal model developed, and thus, there are currently no commercially available norovirus vaccines. Therefore, vaccine development based on VLP (Virus-Like Particles) technology, which is not limited by the possibility of virus proliferation in host cells, is the mainstream.

Virus-like particles (VLPs) are highly complex and sophisticated structures in which viral structural proteins are specifically expressed to have a structure similar to that of wild-type viruses. They have a size of 20 to 100 nm in diameter and can be produced through assembly formation (virion assembly) by specifically expressing structural proteins of viruses whose sequences are already known by using various types of genetic recombinant protein expression systems. Although they have a similar structure and appearance to actual infectious viruses, they cannot proliferate because they do not contain virus-derived genes, but they induce high immunogenicity through high-density and highly ordered conformation. Since it is similar in structure to the wild-type virus, it has the advantage of being able to stimulate both T-cell and B-cell immune pathways in the body. However, since the structure thereof is complex and shows greatly different characteristics depending on the virus, only a few VLPs have been approved as actual commercial vaccines due to reasons such as the need for a complex manufacturing process or low productivity.

Norovirus VLPs can be produced mainly in baculovirus-insect cells, and it is known that VLPs can also be produced in yeast. However, the above methods have the disadvantage of requiring a long manufacturing period, expensive production facilities and high direct manufacturing costs. Considering that most casualties due to norovirus infection occur in developing countries, the production of low-cost vaccines through high-efficiency and rapid production is essential. In order to make this possible, the production of VLPs derived from *E. coli,* which enables high-efficiency and low-cost mass production of viral antigen proteins, is most desirable. However, in the case of *E. coli,* high-efficiency expression of the VP1 protein of each norovirus genotype is possible, but the solubility of the protein is significantly reduced, and as a result, the efficiency of VLP formation is known to be rather low. Therefore, in order to manufacture *E*. coli-derived norovirus VLPs, it is essential to first express a fusion protein with a soluble expression tag to increase the soluble expression rate of the antigen protein.

In a previous study, the inventors of the present invention confirmed that when the RNA interaction domain (RID) including aminoacyl RNA synthetase N-terminal domain (ARSNTD) was fused to the N-terminus of norovirus VP1 protein and expressed, not only was the soluble expression of VP1 protein increased through the action of the intrinsically disordered peptide (IDP) structure of RID, but also the intact VP1 antigen protein was produced with high efficiency through the protein folding induction mechanism of tRNA attached to RID. It was verified that the target protein purified from the above fusion protein enables the rapid production of a large amount of virus-like particles (VLPs) through self-assembly after purification without the need for a separate refolding process (KR Patent No. 1914779 & KR Patent No.2120335).

However, the invention worked well in the case of G II.4, where the structure of the VP1 antigen protein is relatively stable, but it was observed that the soluble expression rate was significantly reduced when applied to the VP1 protein of norovirus of other genotypes. In order to solve this problem, a mutant (hereinafter, m9 RID) was created by substituting all of nine positively charged lysine (K) or arginine (R) residues in the entire RID sequence with alanine (A), thereby lowering the tRNA binding efficiency but being more advantageous in maintaining the intrinsically disordered structure (IDP). When this was applied, it was found to be much more effective in the soluble expression of norovirus VP1 proteins of various genotypes other than G II.4, compared to when the original RID without mutation (wild type RID, wtRID) was attached (KR Patent No.2038876).

In the case of the above m9 RID, the soluble expression can be increased through the action of the IDP structure, but since tRNA binding is excluded, the effect of inducing the folding of the target protein is greatly reduced, and thus, there is a problem in that the soluble expression level is high, but the proportion of soluble aggregates is high and the proportion of the target protein with a proper structure is greatly low. As a result, in the case of m9 RID, although the soluble expression level is high, the final obtainable amount of VLP does not increase proportionally, and thus, the final manufacturing efficiency is rather low.

In order to solve the above problems, the inventors of the present invention performed an analysis of protein motifs using databases such as Pfam, NCBI-CDD and PROSITE and the MOTIF tool, and based on the results, by substituting three or four lysine (K) or arginine (R) residues of a specific sequence of the original RID with alanine (A), a novel mutant m3 RID or m4 RID was prepared that can maintain the folding induction function of the target protein by binding to the original RNA while also maintaining the IDP structure that is essential for enhancing soluble expression.

When the mutant RID was attached to the N-terminus of the VP1 protein of various genotypes of norovirus and expressed, not only was soluble expression greatly improved, but the efficiency of virus-like particle (VLP) production through self-assembly was also greatly increased, thereby completing the present invention.

The present invention demonstrates the advancement of this technique by providing a method for rapidly producing not only GII.4 but also various other genotypes of norovirus VLPs in large quantities in *E. coli* by utilizing a novel mutant RID (m3 RID or m4 RID) that has both the key feature of improved soluble expression efficiency and the ability to induce folding of target proteins by RNA adsorbed to the binding site.

In particular, the present invention is significant in that it enables the development of a VLP-based multivalent vaccine including more genotypes, thereby presenting an appropriate solution for preparing for the threat of new and acute epidemic pathogens due to continuous genetic mutations in the future.

### [Disclosure]

### [Technical Problem]

A one of the objects of the present invention is to provide a fusion protein and a virus-like particle(s) that can be used for the prevention or treatment of infections caused by norovirus.

The main object of the present invention is to present a mutant RID gene combination that can most efficiently express a soluble fusion protein when a norovirus antigen protein is used as a target protein.

### [Technical Solution]

In order to solve the above technical problems, the present invention provides a recombinant expression vector for producing a norovirus vaccine, comprising a polynucleotide encoding: a norovirus antigen protein; and a mutant protein in which 3 or 4 amino acid residues in the sequence of RNA interacting domain (RID) isolated from mammalian lysyl tRNA synthetase (mammalian LysRS) are substituted with other amino acid residues.

In the present invention, the RID may preferably consist of or comprise the amino acid sequences of SEQ ID NO: 2 and/or SEQ ID NO: 3.

The position of the amino acid residues to be substituted in the sequence of the RID of the present invention preferably includes at least any one of the 19^{th}, 23^{rd}, 24^{th}, 30^{th}, 31^{st}, 38^{th} and 40^{th} residues in the amino acid sequence of SEQ ID NO: 3. More preferably, it may include at least any one mutation among K19A, K23A, R24A, K30A, K31A, K38A and K40A in SEQ ID NO: 3. For example, the 23^{rd}, 30^{th} and 31^{st} amino acids in the amino acid sequence of SEQ ID NO: 3 may be substituted, or the 19^{th}, 38^{th} and 40^{th} amino acids may be substituted. According to an exemplary embodiment of the present invention, the mutant protein of the RID may include any one amino acid sequence of the amino acid sequences of SEQ ID NO: 5 to SEQ ID NO: 8.

The norovirus antigen protein of the present invention is preferably an antigen protein including at least any one amino acid sequence selected from the group consisting of SEQ ID NOs: 9 to 13, although the genotype thereof is not limited.

In addition, the present invention provides a host cell transformed by the expression vector. In this case, it is preferably *E. coli.*

According to another exemplary embodiment of the present invention, the present invention provides a fusion protein for a norovirus vaccine, comprising a norovirus antigen protein; and a mutant protein in which 3 or 4 amino acid residues in the sequence of RNA interacting domain (RID) isolated from mammalian lysyl tRNA synthetase (mammalian LysRS) are substituted with other amino acid residues. In the fusion protein of the present invention, at least one linker protein may be positioned between the norovirus antigen protein and the mutant protein of RID.

In addition, the present invention provides a virus-like particle, which may be formed by self-assembly of an antigen protein in which RID is cleaved from the fusion protein. The virus-like particle (VLP) of the present invention may be formed into a size of 30 to 40 nm.

According to another exemplary embodiment of the present invention, provided is a method for soluble production of a norovirus antigen protein (or a method for producing norovirus-like particles), including the steps of a) producing a recombinant expression vector for producing a vaccine, comprising a polynucleotide encoding: a norovirus antigen protein; and a mutant protein in which 3 or 4 amino acid residues in the sequence of RNA interacting domain (RID) isolated from mammalian lysyl tRNA synthetase (mammalian LysRS) are substituted with other amino acid residues; b) producing a transformant by introducing the expression vector into a host cell; and c) culturing the transformant to induce the expression of a recombinant fusion protein and then obtaining the recombinant fusion protein.

In the above production method, the fusion protein may include a tobacco etch virus (TEV) protein between the norovirus antigen protein (the target protein of the present invention) and the RID protein, and after step c), it may include the step of d) cleaving between the RID protein of the prepared fusion protein and the norovirus antigen protein, by using a TEV cleavage enzyme.

In addition, after step d), it may include the step of e) purifying the cleaved antigen protein; and after step e), it may include a step in which the purified protein is self-assembled to form virus-like particles.

In the above production method of the present invention, the RID may consist of or comprise the amino acid sequences of SEQ ID NO: 2 and/or SEQ ID NO: 3, and the substituted amino acid residues may include at least any one of the 19^{th} 23^{rd}, 24^{th}, 30^{th}, 31^{st}, 38^{th} and 40^{th} amino acid residues in the amino acid sequence of SEQ ID NO: 3.

It is most preferable that the mutant protein of the above RID includes any one amino acid sequence of the amino acid sequences of SEQ ID NO: 5 to SEQ ID NO: 8.

In the above production method, the norovirus antigen protein may include at least any one amino acid sequence selected from the group consisting of SEQ ID NOs: 9 to 13.

### [Advantageous Effects]

The peptide including an amino acid sequence encoding a mutant RID protein according to the present invention can significantly enhance the soluble expression of a norovirus antigen protein, which is a target protein, when producing a recombinant protein (fusion protein) in *E. coli.*

In addition, the present invention provides a mutant form of RID that most efficiently folds and improves the yield of final virus-like particle production, when forming a recombinant protein using not only GII. 4, but also various genotypes of norovirus antigen proteins as target proteins.

In addition, the present invention provides a method for rapidly mass-producing not only GII. 4, but also various other genotypes of norovirus VLPs in *E. coli* by utilizing a mutant RID, thereby enabling the development of a VLP-based multivalent vaccine including more genotypes.

### [Description of Drawings]

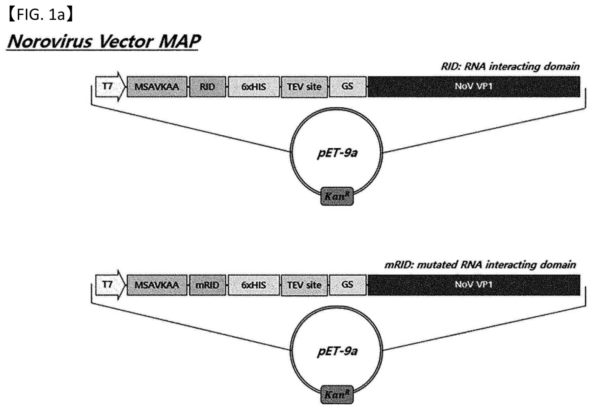

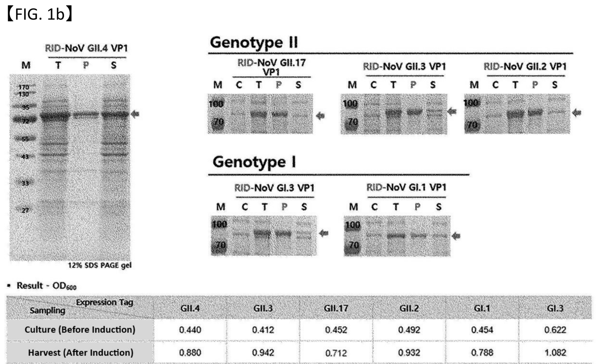

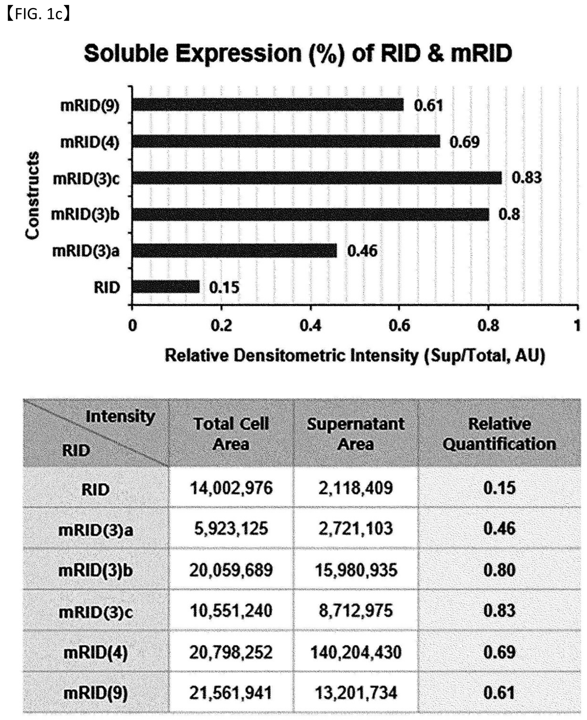

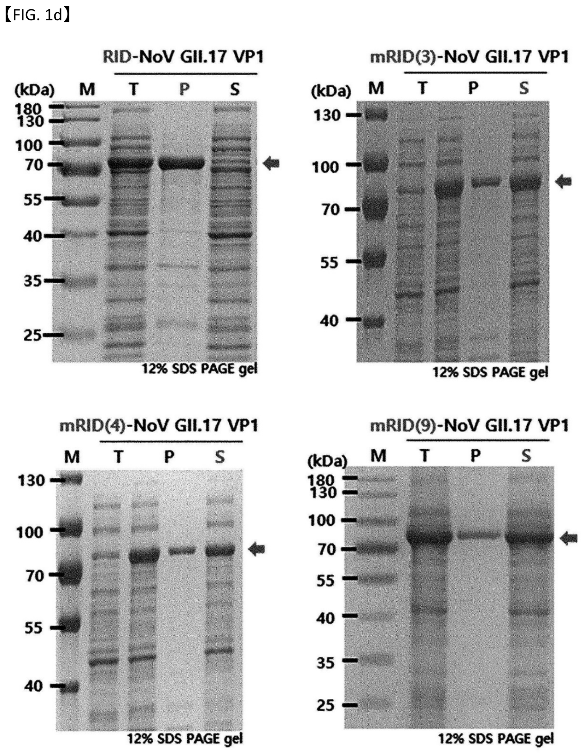

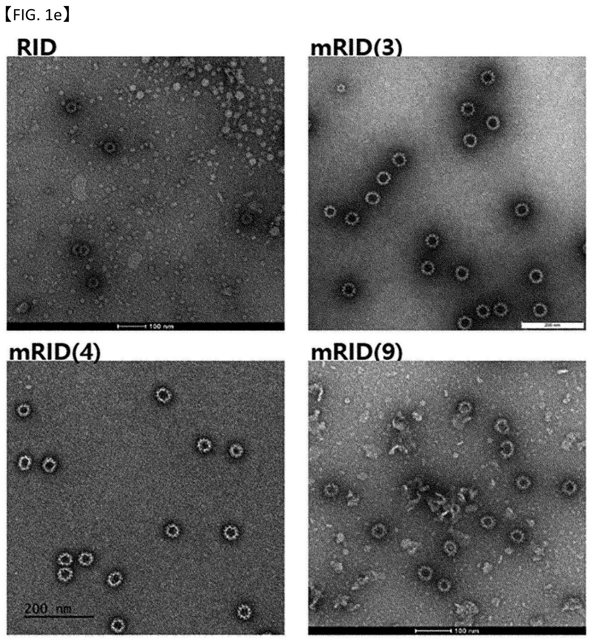

FIG. 1 is a mimetic diagram of a recombinant expression vector for producing norovirus VP1 protein according to an exemplary embodiment of the present invention, and the results of the expression of highly efficient soluble protein according to the expression level and the presence of mutations in RID, which is a fusion partner for enhancing solubility. FIG. 1a is a mimetic diagram showing the structure of a pET9a-based norovirus VP1 protein recombinant expression vector including RID and a mutant RID. FIG. 1b is the result of confirming by SDS-PAGE whether the soluble expression of norovirus VP1 protein combined with RID was achieved according to the genotype of norovirus. FIG. 1c is data comparing the degree of soluble expression of norovirus VP1 protein combined with RID or mutant RID (mRID) prepared according to an exemplary embodiment of the present invention. FIG. 1d is the result of confirming by SDS-PAGE whether the highly efficient soluble expression of norovirus G II.17 VP1 protein combined with RID and a mutant RID was achieved by using an expression vector prepared according to the present invention. FIG. 1e is the result of confirming through transmission electron microscopy (TEM) the overall appearance of the purified norovirus G II.17 VP1 protein-based virus-like particles (VLP) including four RID wild types and mutant types.

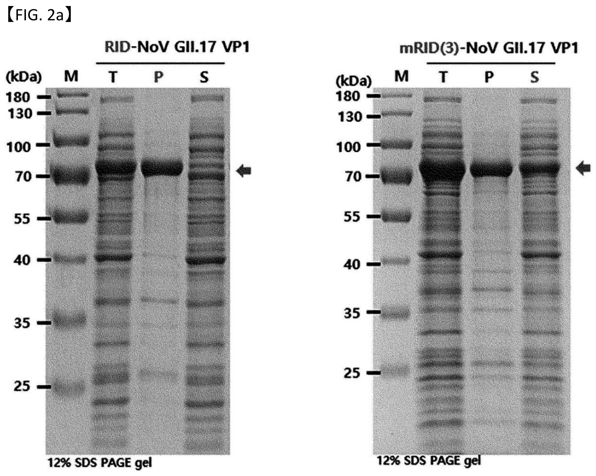

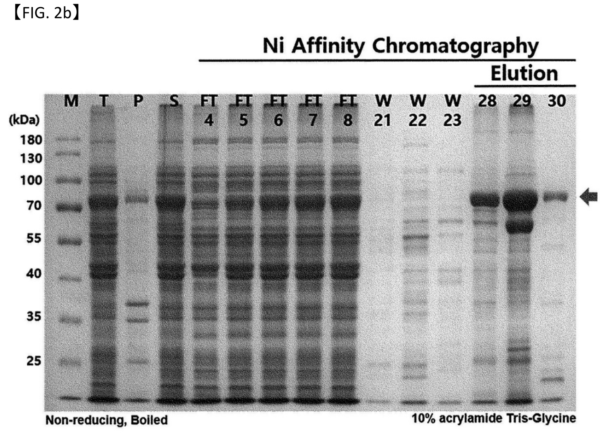

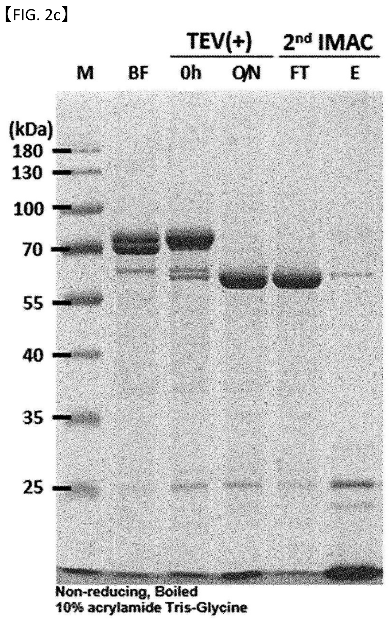

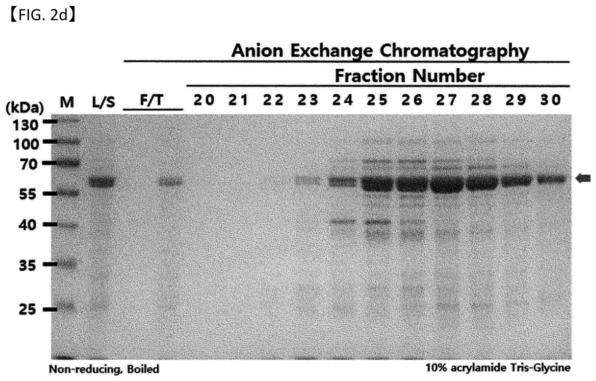

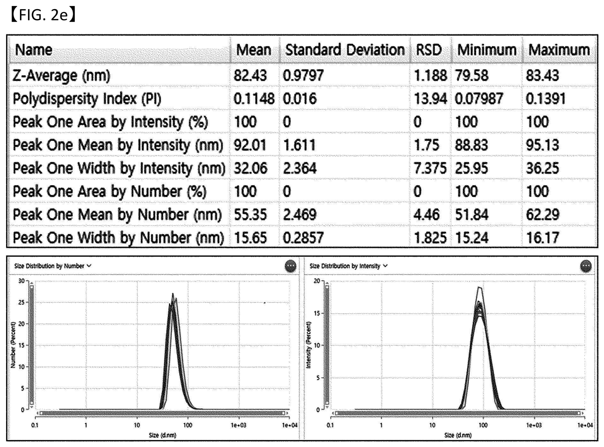

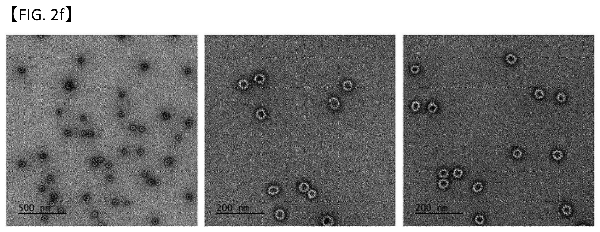

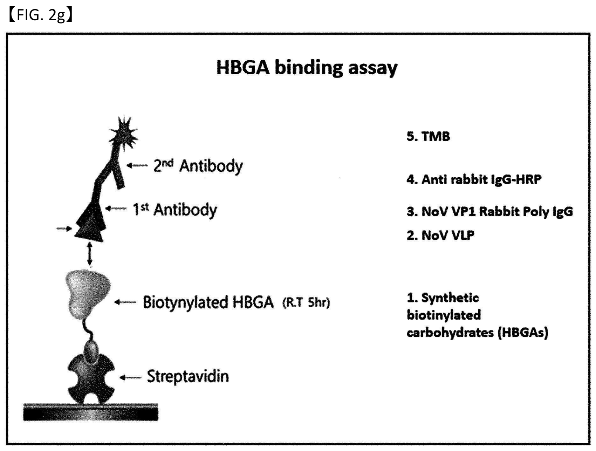

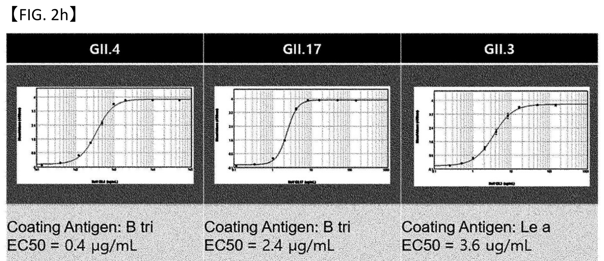

FIG. 2a is the result of confirming the effect of the m3c mutation of RID on the soluble expression of the norovirus GII.17 VP1 fusion protein according to an exemplary embodiment of the present invention. Unlike the norovirus GII.4 VP1 protein, the norovirus GII.17 VP1 protein mostly showed an insoluble expression pattern when RID was fused to the N-terminus thereof, whereas most were expressed to be soluble when m3c was fused to the N-terminus thereof, as confirmed through SDS-PAGE. FIG. 2b is the result of confirming the protein separation pattern when the norovirus GII.17 VP1 fusion protein including m3c among the mutant RIDs expressed according to an exemplary embodiment of the present invention was purified through nickel affinity chromatography, as confirmed by SDS-PAGE. FIG. 2c is the result of confirming the series of processes from the cleavage using TEV enzyme of the RID (EE domain + m3c) - norovirus GII.17 VP1 fusion protein eluted from nickel affinity chromatography to the acquisition of the flow-through (FT) fraction eluted from the subsequent nickel affinity chromatography, as confirmed by SDS-PAGE. FIG. 2d is the result of confirming the purification pattern when the norovirus GII.17 VP1 protein eluted from nickel affinity chromatography was purified through ion resin exchange chromatography, as confirmed by SDS-PAGE. FIG. 2e is the result of analyzing the diameter distribution of virus-like particles (VLPs) prepared after the self-assembly step of purified norovirus GII.17 VP1 protein through dynamic light scattering (DLS). FIG. 2f is the result of confirming the overall appearance of the prepared norovirus GII.17 virus-like particles through transmission electron microscopy (TEM). FIG. 2g is a mimetic diagram showing a binding constitution for HBGA binding affinity analysis according to an exemplary embodiment of the present invention. FIG. 2h is the result of analyzing the degree of binding of GII.4, GII.17 and GII.3 VLP proteins to Blood type B (tri), Blood type B (tri) and Le a (H type1), respectively, and the corresponding binding affinity of each VLP is expressed as EC50.

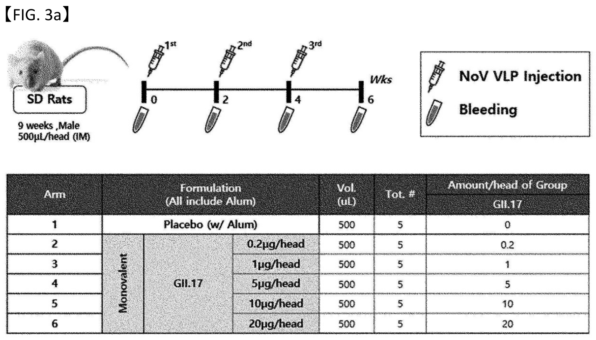

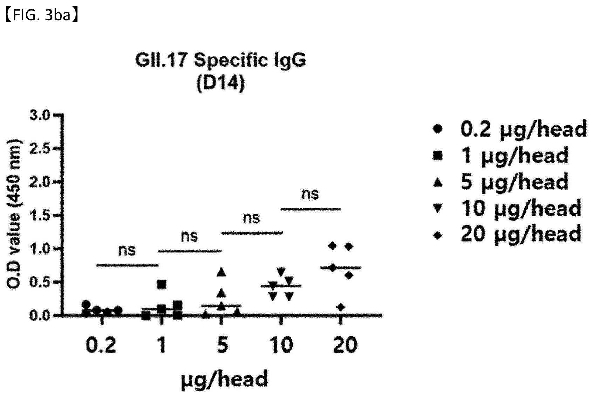

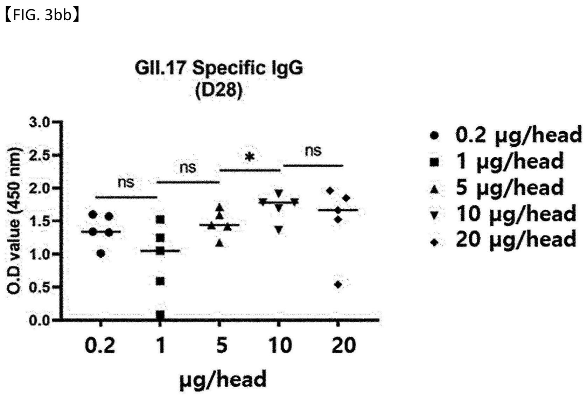

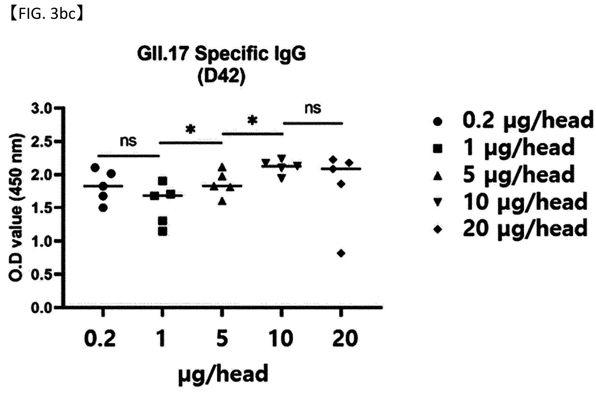

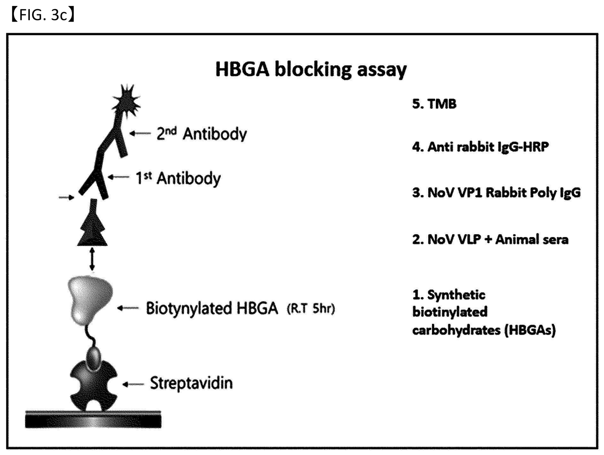

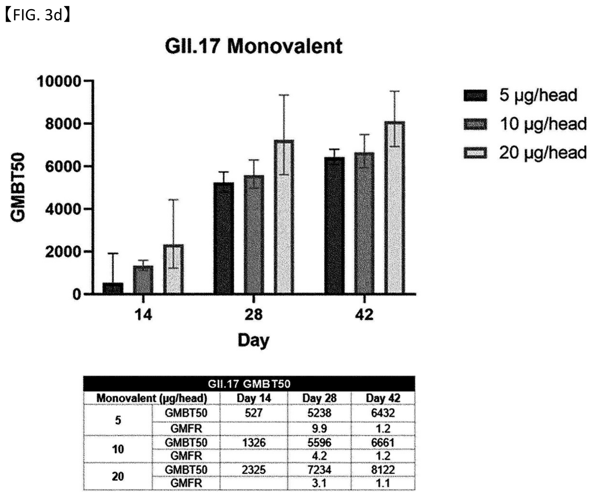

FIG. 3a is a mimetic diagram showing an experimental process conducted to measure the specific antibody-inducing ability of GII. 17 virus-like particles (VLPs) prepared according to an exemplary embodiment of the present invention. FIGS. 3ba, 3bb and 3bc are the results of measuring the degree of specific antibody induction according to the administered dose in blood serum collected after administering the virus-like particles (NoV GII. 17 VLPs) according to the present invention once, twice and three times, respectively, by enzyme-linked immunosorbent assay (ELISA). FIG. 3c is a mimetic diagram showing a binding constitution for analyzing the binding inhibition efficacy between the immune serum and the GII. 17 VLP antigen (VLP composed of an antigen protein purified after the cleavage of RID (EE domain + m3c)-NoV GII. 17 VP1 fusion protein of the present invention, Nov VLP in FIG. 3c) and HBGA. FIG. 3d is the result of confirming the difference in HBGA binding inhibition ability between the first, second and third inoculation groups after inoculating each dose of norovirus GII.17 VLP according to an exemplary embodiment of the present invention.

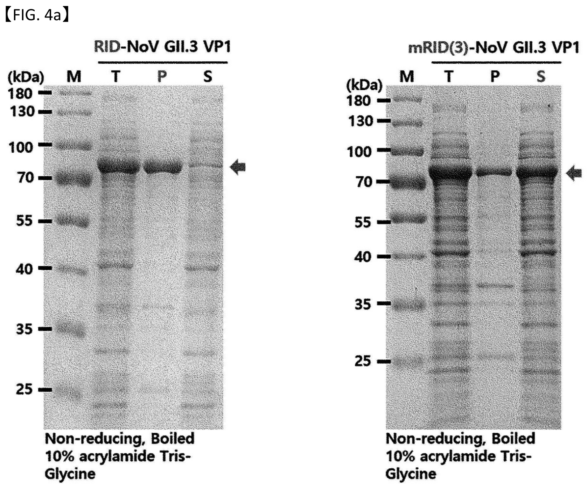

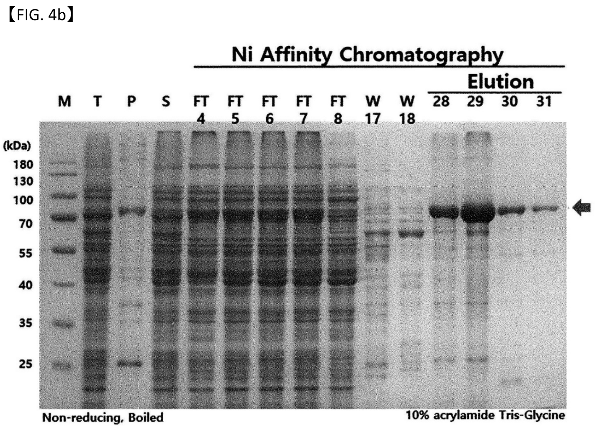

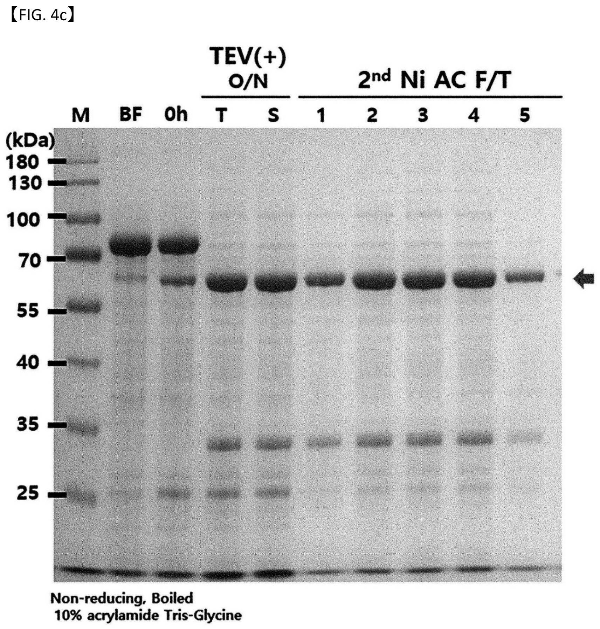

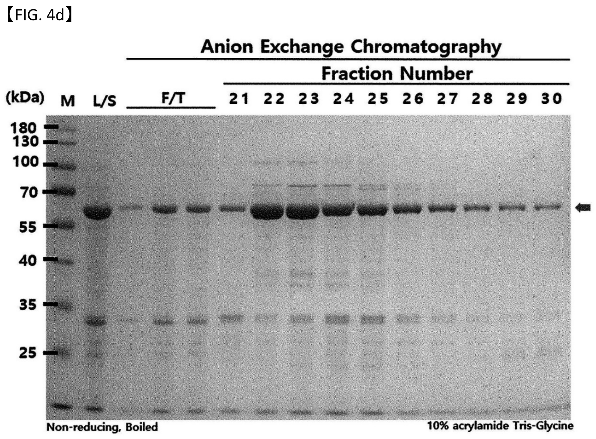

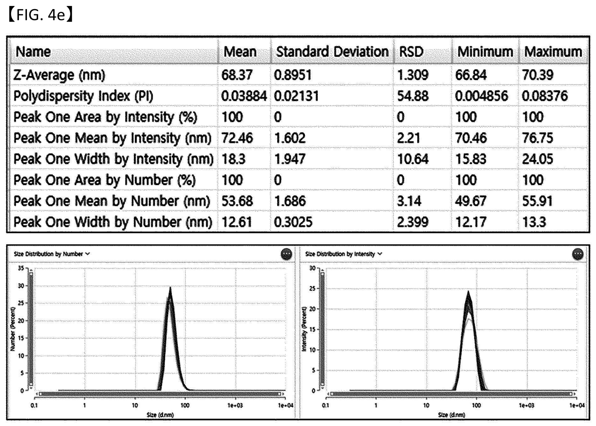

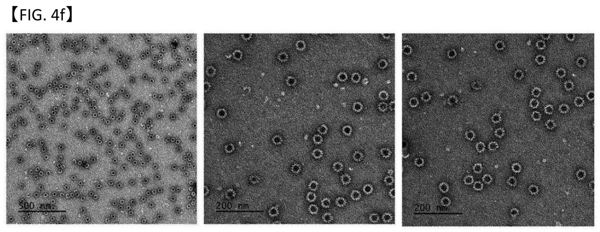

FIG. 4a is the result of confirming the effect on the soluble expression of norovirus GII.3 VP1 protein according to the presence of mutation of RID according to an exemplary embodiment of the present invention. In the case of proteins fused with RID at the N-terminus, most showed an insoluble expression pattern, whereas when RID (EE domain + m3c) was fused to the N-terminus and expressed, most were expressed to be soluble, as confirmed through SDS-PAGE. FIG. 4b is the result of confirming the protein separation pattern when the norovirus GII.3 VP1 protein including m3c among the mutant RIDs expressed according to an exemplary embodiment of the present invention was purified through nickel affinity chromatography, as confirmed through SDS-PAGE. FIG. 4c is the result of confirming a series of processes from the cleavage of RID(EE domain+m3c)-norovirus GII.3 VP1 fusion protein eluted from nickel affinity chromatography using TEV enzyme to the acquisition of the flow-through (FT) fraction eluted from the subsequent nickel affinity chromatography, as confirmed by SDS-PAGE. FIG. 4d is the result of confirming the purification pattern of norovirus GII.3 VP1 protein eluted from nickel affinity chromatography through ion resin exchange chromatography, as confirmed by SDS-PAGE. FIG. 4e is the result of analyzing the diameter distribution of virus-like particles (VLPs) prepared after the self-assembly step using the purified norovirus GII.3 protein through dynamic light scattering (DLS). FIG. 4f is the result of confirming the overall appearance of the prepared norovirus GII.3 virus-like particles through transmission electron microscopy (TEM).

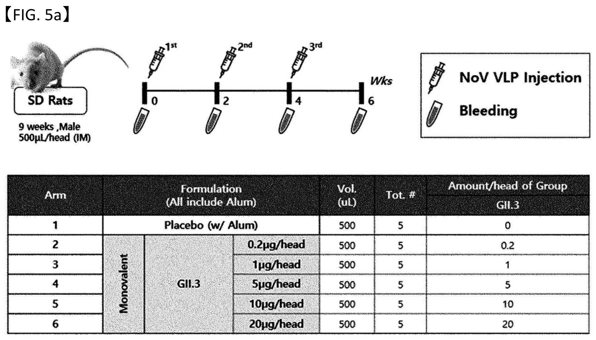

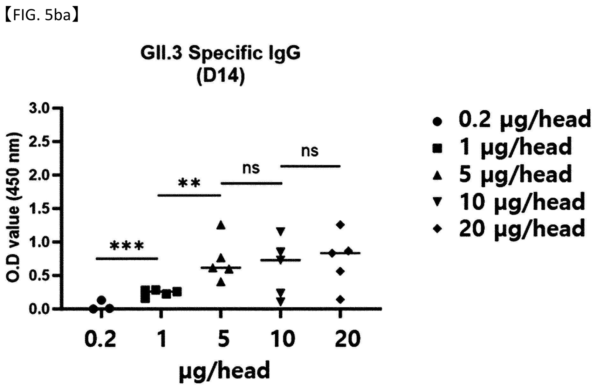

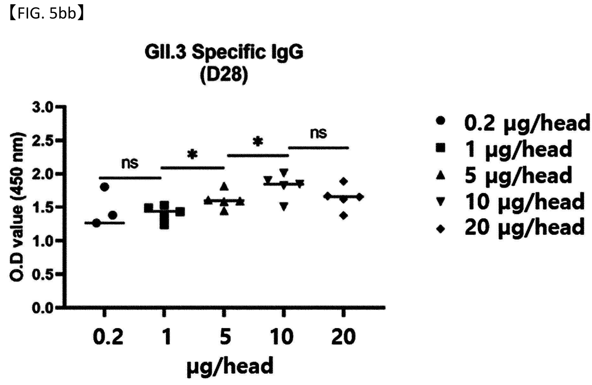

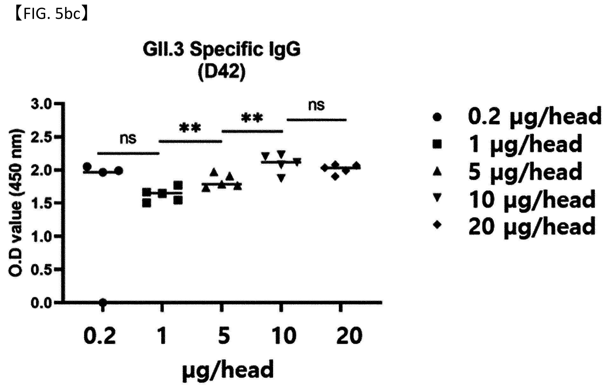

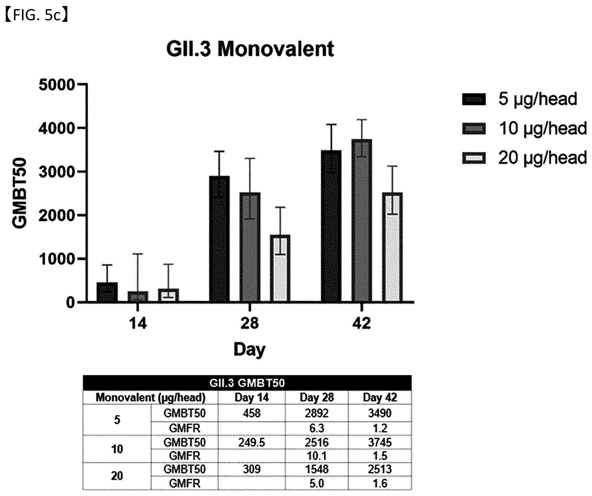

FIG. 5a is a mimetic diagram showing the experimental process conducted to measure the specific antibody induction ability of the GII.3 virus-like particles (VLP) prepared according to an exemplary embodiment of the present invention. FIGS. 5ba, 5bb and 5bc are the results of measuring the degree of specific antibody induction according to the administered dose in the blood serum collected after administering the virus-like particles (NoV GII.3 VLP) according to the present invention once, twice and three times, respectively, by enzyme-linked immunosorbent assay (ELISA). FIG. 5c is the result of confirming the difference in HBGA binding inhibition ability between the first, second and third inoculation groups after inoculating each dose of the norovirus GII.3 VLP according to an exemplary embodiment of the present invention.

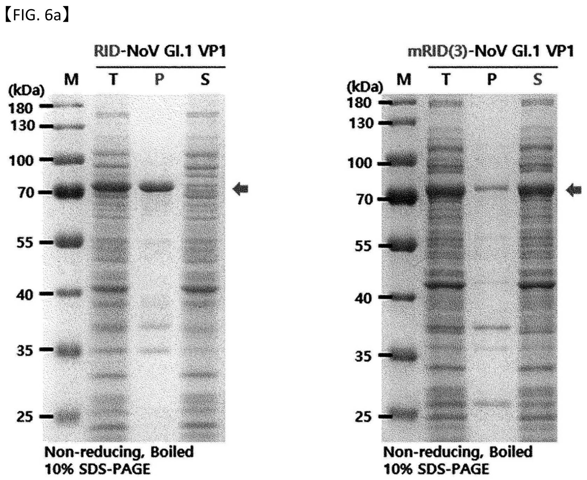

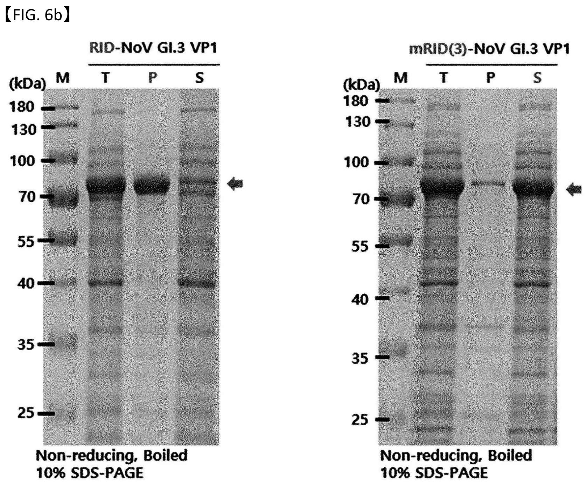

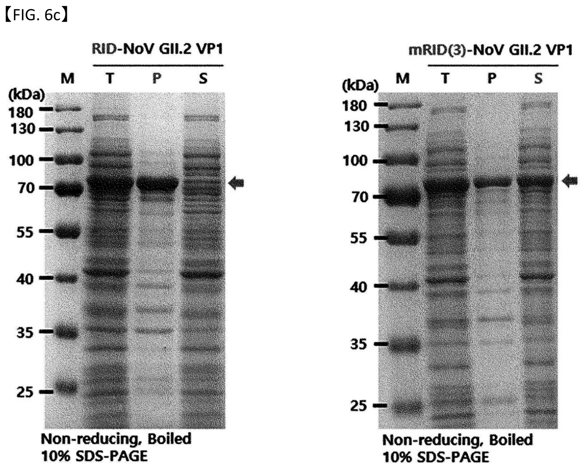

FIG. 6a is the result of confirming the effect on the soluble expression of a norovirus GI.1 VP1 protein-fused protein according to the expression level and the presence of the m3c mutation in RID, which is a solubility-enhancing fusion partner. In the case of proteins fused with RID at the N-terminus, most showed an insoluble expression pattern, whereas when RID (EE domain + m3c) was fused to the N-terminus and expressed, most were expressed to be soluble, as confirmed through SDS-PAGE. FIG. 6b is the result of confirming the effect on the soluble expression of a norovirus GI.3 VP1 fusion protein according to the presence of a mutation in RID (particularly, the SE domain in RID) according to an exemplary embodiment of the present invention. In the case of proteins fused with RID at the N-terminus, most showed an insoluble expression pattern, whereas when RID (EE domain + m3c) was fused to the N-terminus and expressed, most were expressed to be soluble, as confirmed through SDS-PAGE. FIG. 6c is a result of confirming the effect on the soluble expression of the fusion protein of the norovirus GII.2 VP1 protein and the target protein according to the expression level and the presence of the m3c mutation of RID, which is a solubility (or water-solubility) enhancing fusion partner, according to an exemplary embodiment of the present invention. In the case of proteins fused with RID at the N-terminus, most showed an insoluble expression pattern, whereas when RID (EE domain + m3c) was fused to the N-terminus and expressed, most were expressed to be soluble, as confirmed through SDS-PAGE.

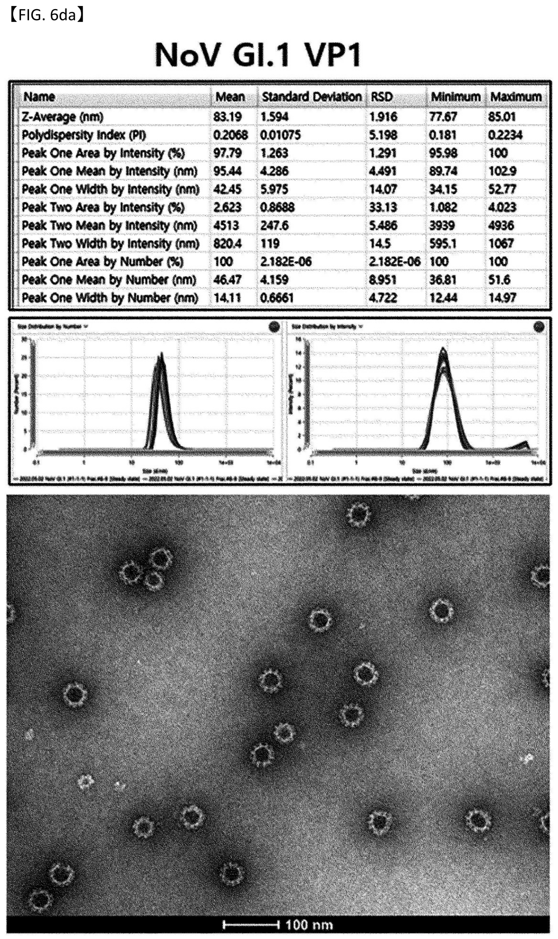

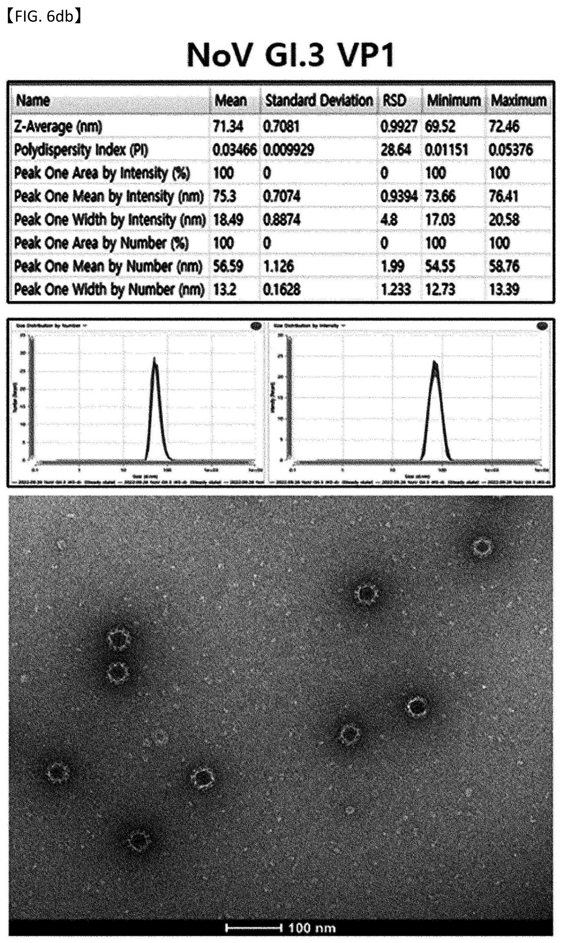

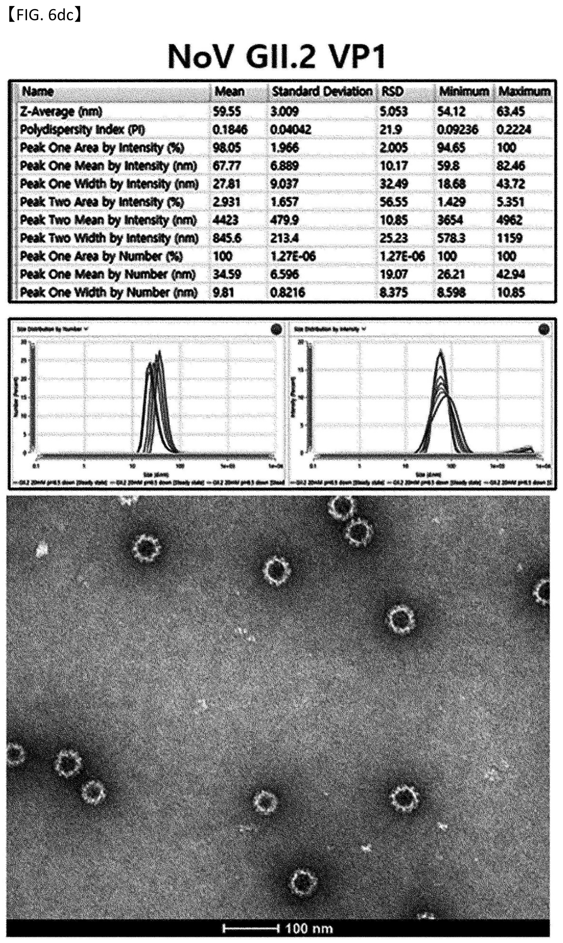

FIG. 6da is the results of confirming the size distribution (top of FIG. 6da) and particle formation of the norovirus GI.1 VLP particles prepared only with the finally purified norovirus GI.1 VP1 protein after the expression of the fusion protein with RID (EE domain + m3c) according to the present invention through dynamic light scattering (DLS) and transmission electron microscopy (TEM), respectively. FIG. 6db is an image confirming the size distribution (top of FIG. 6db) and particle formation of norovirus GI.1 VLP particles prepared only with the finally purified norovirus GI.3 VP1 protein after expression of the fusion protein with RID (EE domain + m3c) according to the present invention, and FIG. 6dc is an image confirming the size distribution (top of FIG. 6dc) and particle formation of norovirus GII.2 VLP particles prepared only with the finally purified norovirus GII.2 VP1 protein after expression of the fusion protein with RID (EE domain + m3c) according to the present invention.

### [Modes of the Invention]

Hereinafter, preferred exemplary embodiments of the present invention will be described in detail. The advantages and features of the present invention will become clear with reference to the exemplary embodiments described below that achieve the same. However, the present invention is not limited to the exemplary embodiments disclosed below, but can be implemented in various different forms, and these exemplary embodiments are provided only to make the disclosure of the present invention complete and to fully inform those skilled in the art of the present invention of the scope of the invention, and the present invention is defined only by the scope of the claims.

Like reference numerals throughout the specification refer to like elements. Unless otherwise defined, all terms (including technical and scientific terms) used in the present specification may be used in meanings that can be commonly understood by those skilled in the art of the present invention. In addition, terms defined in commonly used dictionaries shall not be overly interpreted unless explicitly specifically defined. The terms used in the present specification are for the purpose of describing the exemplary embodiments and are not intended to limit the present invention. In the present specification, singular forms also include plural forms unless specifically stated in the phrase.

As used herein, the term "target protein" refers to a protein that a person skilled in the art intends to mass-produce, and a polynucleotide encoding the target protein is inserted into the recombinant expression vector of the present invention and can be expressed in a host cell.

According to an exemplary embodiment of the present invention, the target protein includes a viral antigen protein, an antibody, a cell receptor, an enzyme, a structural protein, a serum and a cell protein. According to a preferred exemplary embodiment of the present invention, the viral antigen protein is a norovirus antigen protein.

The norovirus antigen protein of the present invention may include GI and GII among the norovirus types, and more preferably, it may include norovirus GI.1, GI.3, GII.17, GII.2 and GII.3 VP1 antigen proteins. The norovirus VP1 antigen protein of the present invention may consist of an amino acid sequence shown in SEQ ID NOs: 9 to 13, but the present invention is not limited thereto.

As used herein, the term "RID", "RNA interacting domain" or "N terminal appended RNA binding domain of Lysyl tRNA synthetase (ARSNTD)" refers to a unique N-terminal extension region of mammalian LysRS involved in the interaction between RNA and other proteins.

According to an exemplary embodiment of the present invention, the RID protein can be used as a fusion partner for increasing the expression level or solubility of the target protein.

According to an exemplary embodiment of the present invention, the RID is an amino acid sequence (wild type) of an N-terminal domain (hRID, hLysRS N-terminal appended RNA interacting domain) isolated from mammalian lysyl tRNA synthetase (hLysRS, human lysyl tRNA synthetase), and the mutant RID includes mutant sequences in which one or more positively charged amino acid residues in the sequence of the RID are substituted with other amino acid residues.

According to a preferred exemplary embodiment of the present invention, the RID protein according to the present invention includes an EE domain (SEQ ID NO: 2, MSAVKAA) used to increase the expression level of the target protein and an SE domain (SEQ ID NO: 3) used to enhance soluble expression by inducing folding of the target protein.

The RID protein used in the present invention may be mutated. As used herein, the term "mutation" means that one or more residues in an amino acid sequence are substituted with other amino acid residues, some residues in an amino acid sequence are deleted, or a specific amino acid residue is inserted. In the present invention, "mutation" is used interchangeably with "point mutation" or "site directed mutation", and means a mutation in which an amino acid residue at a specific position is substituted with another amino acid residue.

In the present specification, the amino acid sequence of "RID" may include the amino acid sequences of SEQ ID NO: 2 and/or SEQ ID NO: 3.

According to an exemplary embodiment of the present invention, it is preferred that the mutation of RID is a substitution of 3 to 4 amino acid residues on the SE domain. For example, 3 to 4 amino acid residues may be substituted in the amino acid sequence of SEQ ID NO: 3 (the SE domain of the RID in the present invention). According to a preferred exemplary embodiment of the present invention, in the amino acid sequence of the SE domain of the present invention (SEQ ID NO: 3), the substituted amino acid residues may include at least any one of the 19^{th}, 23^{rd}, 24^{th}, 30^{th}, 31^{st}, 38^{th} and 40^{th} amino acids. For example, the 23^{rd}, 30^{th} and 31^{st} amino acids in the amino acid sequence of SEQ ID NO: 3 may be substituted, or the 19^{th}, 38^{th} and 40^{th} amino acids may be substituted. It is preferred that the substitution target includes at least one of K19A, K23A, R24A, K30A, K31A, K38A and K40A. More preferably, in the amino acid sequence of the RID SE domain (SEQ ID NO: 3), any one of the 23^{rd}, 24^{th}, 30^{th} and 31^{st} amino acid residues (e.g., K23, R24, K30 and K31) may be substituted. That is, according to an exemplary embodiment of the present invention, the amino acid sequence of the RID mutated according to the present invention may further include any one of the sequences of SEQ ID NOs: 5 to 8 in addition to SEQ ID NO: 2.

The names of the sequences used in the present invention are organized as shown in Table 1 below.

**[Table 1]**

| Name | Amino Acid Sequence | Content |
|---|---|---|
| RID EE domain | SEQ ID NO: 2 | Inducing an increase in expression level |
| RID SE domain | SEQ ID NO: 3 | Folding-induced mutation pre-domain |
| RID SE domain mn (n is a number) | - | RID SE domain with n amino acid residues substituted |
| RID SE domain m9 | SEQ ID NO: 4 | RID SE domain with 9 amino acids substituted |
| RID SE domain m3a | SEQ ID NO: 5 | RID SE domain a with 3 amino acids substituted (K19A/K38A/K40A) |
| RID SE domain m3b | SEQ ID NO: 6 | RID SE domain b with 3 amino acids substituted (K19A/K23A/R24A) |
| RID SE domain m3c | SEQ ID NO: 7 | RID SE domain c with 3 amino acids substituted (K23A/K30A/K31A) |
| RID SE domain m4 | SEQ ID NO: 8 | RID SE domain with 4 amino acids substituted (K23A/R24A/K30A/K31A) |

In the present invention, the EE domain may include an amino acid sequence of SEQ ID NO: 2 or a part of the sequence thereof.

According to an exemplary embodiment of the present invention, when a norovirus antigen protein is a target protein in the production of a fusion protein of the present invention, the solubility of the fusion protein was shown to be higher when a mutated RID protein (a protein consisting of an amino acid sequence of any one of SEQ ID NOs: 4 to 8 and an amino acid sequence of SEQ ID NO: 2) was combined than when a non-mutated RID protein was combined (refer to FIG. 1c). In addition, when the m3 or m4 protein with three or four amino acid residues substituted was combined with the norovirus antigen protein, protein folding occurred better than the m9 protein with nine mutations in the SE domain among the RIDs (refer to FIG. 1e), and when the RID including m3 or m4 with three or four amino acid residues substituted in the SE domain sequence was combined with the norovirus antigen protein, the effect of increasing the yield of soluble expression was observed, and moreover, the efficiency of VLP formation could be improved through more accurate folding induction.

As used herein, the term "expression vector" is a linear or circular DNA molecule consisting of a fragment encoding a target protein which is operably linked to an additional fragment provided for transcription of the expression vector. Such an additional fragment includes a promoter and a termination sequence. The expression vector also includes one or more replication origins, one or more selection markers and the like. The expression vector generally contains elements derived from plasmid or viral DNA, or both. As used herein, the term "operably linked" indicates that the fragments are arranged such that transcription is initiated from the promoter and proceeds through the coding sequence to the termination sequence.

In the expression vector according to the present invention, the expression vector may be a plasmid, a viral vector, a phage particle or a genomic insert. The expression vector may be replicated or integrated into the genome of the host cell independently of the genome of the host cell after being transformed into the host cell.

According to an exemplary embodiment of the present invention, the polynucleotide may additionally encode a "linker", and at least one linker protein may be positioned between the target protein and the RNA interacting domain (RID) protein, respectively.

According to a preferred exemplary embodiment of the present invention, the sequence of the fusion protein of the present invention, particularly the sequence of the target protein, may include a TEV-specific cleavage sequence (TEV-specific cleavage site) for the cleavage of RID.

In the recombinant expression vector for vaccine production of the present invention, the protein cleavage enzyme may be TEV.

In addition, the present invention provides a host cell which is transformed by the expression vector.

As used herein, the term "transformation" or "introduction" means introducing DNA into a host such that the DNA becomes replicable as an extrachromosomal element or by the completion of chromosomal integration. The method for transforming the expression vector according to the present invention may include electroporation, calcium phosphate (CaPO₄) method, calcium chloride (CaCl₂) method, microinjection, polyethylene glycol (PEG) method, DEAE-dextran method, cationic liposome method or lithium acetate-DMSO method, but the present invention is not limited thereto.

In addition, the present invention provides a fusion protein including a target protein and a RID protein, and in the present invention, the RNA interacting domain (RID) protein may include a mutant RID in which 3 (m3) to 4 (m4) amino acid residues of the SE domain are substituted.

As used herein, the term "fusion protein" refers to a protein in which another protein is linked to the N-terminus or C-terminus of a target protein sequence or another amino acid sequence is added. In addition, the fusion protein in the present invention may be a vaccine produced by the recombinant expression vector of the present invention. According to an exemplary embodiment of the present invention, the fusion protein of the present invention is a protein in which a mutant RID and a target protein (norovirus antigen protein) are fused.

According to an exemplary embodiment of the present invention, a fusion protein can be formed by using the recombinant expression vector, and the fusion protein can be used as a vaccine by itself or through any additional process.

In addition, the present invention provides a virus-like particle (VLP) in which a plurality of antigen proteins in which the RID is cleaved from the fusion protein are combined. The virus-like particle according to the present invention can be formed as a purified antigen protein (e.g., norovirus VP1 antigen protein) by cleaving the mutant RID of the fusion protein during the formation process.

Hereinafter, the present invention will be described in more detail through examples.

These examples are only for illustrating the present invention, and it will be apparent to those skilled in the art that the scope of the present invention is not to be construed as being limited by these examples.

### [Example 1]

### Preparation of norovirus fusion protein including mutant RID

### <Example 1-1>

In order to produce virus-like particles composed of norovirus GII.17 VP1 protein, the VP1 gene derived from norovirus GII.17 protein (Norovirus Hu/GII.17/HKG/2014/CUHK-NS-405, NCBI access number: KP902566.1) was used. VP1, which is an antigen of norovirus, consists of 539 amino acids, and the molecular weight of the protein is approximately 59.2 kDa. Based on the protein sequence, a gene sequence synthesized after codon optimization in *E. coli* was prepared (SEQ ID NO: 9) (Request for gene synthesis to Bionics Co., Ltd.).

In this example, an RID EE domain peptide (MSAVKAA (SEQ ID NO: 2)) and a RID SE domain peptide (SEQ ID NO: 3) that have the effect of increasing expression level and increasing solubility at the N-terminus of the norovirus GII.17 VP1 protein were used. Additionally, in order to confirm the effect by the RID mutation, mutant RID SE domain peptides that are more than 50% similar to SEQ ID NO: 3 were used instead of the RID SE domain peptide, which included a mutant RID SE domain peptide (SEQ ID NO: 5) including mutations of K19A, K38A and K40A, a mutant RID SE domain peptide (SEQ ID NO: 7) including mutations of K23A, K30A and K31A, or nine-mutation RID SE domain peptide (SEQ ID NO: 4), respectively. In this example, recombinant proteins fused with each mutant SE domain were produced, and the soluble expression pattern was verified.

In addition, ENLYFQG/S is known as the sequence of the TEV cleavage site, and a peptide having an amino acid sequence of (GaSb)ₙ (a≥1, b≥1, n≥1) was designed for higher efficiency cleavage and inserted between the TEV cleavage sequence (ENLYFQ) and the target protein (norovirus antigen protein) sequence. A mimetic diagram of the fusion protein expression vector produced in this example is as shown in FIG. 1a.

The gene of the above sequence was inserted into the MCS of the expression vector pET-9a (Novagene, 69431-3CN) (SEQ ID NO: 1) using the cleavage enzymes Ndel and BamHI, and the sequence of the final recombinant expression vector is as follows.

EE domain (MSAVKAA)-SE domain-GT-6xHIS-TEV-GS-NoV GII.17 VP1
[(SEQ ID NO: 2)-(SEQ ID NO: 3)-GT-6xHIS-TEV-GS-(SEQ ID NO: 9)]

:MSAVKAA-
AAVQAAEVKVDGSEPKLSKNELKRRLKAEKKVAEKEAKQKELSEKQLSQATAAATNHTTDNGV
GPEEESV-GT-HHHHHH-ENLYFQ-GS-
KMASNDAAPSNDGAAGLVPEGNNETLPLEPVAGAAIAAPVTGQNNIIDPWIRTNFVQAPNG
EFTVSPRNSPGEILLNLELGPDLNPYLAHLSRMYNGYAGGVEVQVLLAGNAFTAGKILFAAVPP
NFPVEFLSPAQITMLPHLIVDVRTLEPIMIPLPDVRNTFFHYSNQPNSRMRLVAMLYTPLRSNG
SGDDVFTVSCRVLTRPTPDFEFTYLVPPSVESKTKPFSLPILTLSELTNSRFPVPIDSLFTAQNNVL
QVQCQNGRCTLDGELQGTTQLLPSGICAFRGRVTAQINQRDRWHMQLQNLNGTTYDPTDD
VPAPLGTPDFKGVVFGMVSQRNVGNDAPGSTRAQQAWVSTYSPQFVPKLGSVNLRISDNDD
FQFQPTKFTPVGVNDDDDGHPFRQWELPNYSGELTLNMNLAPPVAPNFPGEQLLFFRSFVPC
SGGYNQGIIDCLIPQEWIQHFYQESAPSQSDVALIRYVNPDTGRTLFEAKLHRSGYITVAHSGDY
PLVVPANGHFRFDSWVNQFYSLAPMGTGNGRRRAQ

The prepared complex above was named as "RID-NoV GII.17 VP1."

MSAVKAA-m3a-GT-6xHIS-TEV-GS-NoV GII.17 VP1 [(SEQ ID NO. 2)-(SEQ ID NO. 5)-GT-6xHIS-TEV-GS-(SEQ ID NO. 9)]

:MSAVKAA-
AAVQAAEVKVDGSEPKLSANELKRRLKAEKKVAEKEAAQAELSEKQLSQATAAATNHTTDNGV

GPEEESV
-GT-HHHHHH-ENLYFQ-GS-
KMASNDAAPSNDGAAGLVPEGNNETLPLEPVAGAAIAAPVTGQNNIIDPWIRTNFVQAPNG
EFTVSPRNSPGEILLNLELGPDLNPYLAHLSRMYNGYAGGVEVQVLLAGNAFTAGKILFAAVPP
NFPVEFLSPAQITMLPHLIVDVRTLEPIMIPLPDVRNTFFHYSNQPNSRMRLVAMLYTPLRSNG
SGDDVFTVSCRVLTRPTPDFEFTYLVPPSVESKTKPFSLPILTLSELTNSRFPVPIDSLFTAQNNVL
QVQCQNGRCTLDGELQGTTQLLPSGICAFRGRVTAQINQRDRWHMQLQNLNGTTYDPTDD
VPAPLGTPDFKGVVFGMVSQRNVGNDAPGSTRAQQAWVSTYSPQFVPKLGSVNLRISDNDD
FQFQPTKFTPVGVNDDDDGHPFRQWELPNYSGELTLNMNLAPPVAPNFPGEQLLFFRSFVPC
SGGYNQGIIDCLIPQEWIQHFYQESAPSQSDVALIRYVNPDTGRTLFEAKLHRSGYITVAHSGDY
PLVVPANGHFRFDSWVNQFYSLAPMGTGNGRRRAQ

The prepared complex above was named as "RID(EE domain+m3a)-NoV GII.17 VP1."

MSAVKAA-m3c-GT-6xHIS-TEV-GS-NoV GII.17 VP1 [(SEQ ID NO. 2)-(SEQ ID NO. 7)- GT-6xHIS-TEV-GS-(SEQ ID NO. 9)]

:MSAVKAA-
AAVQAAEVKVDGSEPKLSKNELARRLKAEAAVAEKEAKQKELSEKQLSQATAAATNHTTDNGV
GPEEESV
-GT-HHHHHH-ENLYFQ-GS-
KMASNDAAPSNDGAAGLVPEGNNETLPLEPVAGAAIAAPVTGQNNIIDPWIRTNFVQAPNG
EFTVSPRNSPGEILLNLELGPDLNPYLAHLSRMYNGYAGGVEVQVLLAGNAFTAGKILFAAVPP
NFPVEFLSPAQITMLPHLIVDVRTLEPIMIPLPDVRNTFFHYSNQPNSRMRLVAMLYTPLRSNG
SGDDVFTVSCRVLTRPTPDFEFTYLVPPSVESKTKPFSLPILTLSELTNSRFPVPIDSLFTAQNNVL
QVQCQNGRCTLDGELQGTTQLLPSGICAFRGRVTAQINQRDRWHMQLQNLNGTTYDPTDD
VPAPLGTPDFKGVVFGMVSQRNVGNDAPGSTRAQQAWVSTYSPQFVPKLGSVNLRISDNDD
FQFQPTKFTPVGVNDDDDGHPFRQWELPNYSGELTLNMNLAPPVAPNFPGEQLLFFRSFVPC
SGGYNQGIIDCLIPQEWIQHFYQESAPSQSDVALIRYVNPDTGRTLFEAKLHRSGYITVAHSGDY
PLVVPANGHFRFDSWVNQFYSLAPMGTGNGRRRAQ

The prepared complex above was named as "RID(EE domain+m3c)-NoV GII.17 VP1."

MSAVKAA-m9 RID-GT-6xHIS-TEV-GS-NoV GII.17 VP1 [(SEQ ID NO. 2)-(SEQ ID NO. 4)- GT-6xHIS-TEV-GS- SEQ ID NO. 9)]

:MSAVKAA-
AAVQAAEVKVDGSEPKLSANELAARLAAEAAVAEAEAAQAELSEKQLSQATAAATNHTTDNG
VGPEEESV
-GT-HHHHHH-ENLYFQ-GS-
KMASNDAAPSNDGAAGLVPEGNNETLPLEPVAGAAIAAPVTGQNNIIDPWIRTNFVQAPNG
EFTVSPRNSPGEILLNLELGPDLNPYLAHLSRMYNGYAGGVEVQVLLAGNAFTAGKILFAAVPP

NFPVEFLSPAQITMLPHLIVDVRTLEPIMIPLPDVRNTFFHYSNQPNSRMRLVAMLYTPLRSNG
SGDDVFTVSCRVLTRPTPDFEFTYLVPPSVESKTKPFSLPILTLSELTNSRFPVPIDSLFTAQNNVL
QVQCQNGRCTLDGELQGTTQLLPSGICAFRGRVTAQINQRDRWHMQLQNLNGTTYDPTDD
VPAPLGTPDFKGVVFGMVSQRNVGNDAPGSTRAQQAWVSTYSPQFVPKLGSVNLRISDNDD
FQFQPTKFTPVGVNDDDDGHPFRQWELPNYSGELTLNMNLAPPVAPNFPGEQLLFFRSFVPC
SGGYNQGIIDCLIPQEWIQHFYQESAPSQSDVALIRYVNPDTGRTLFEAKLHRSGYITVAHSGDY
PLVVPANGHFRFDSWVNQFYSLAPMGTGNGRRRAQ

The prepared complex above was named as "RID(EE domain+m9)-NoV Gll.17 VP1."

### <Example 1-2>

The expression vector produced in Example 1-1 above was transformed into HMS174 competent cells (Novagen (RecA mutation in a K-12 strain, Cat: 69453-3)). All transformed *E. coli* were cultured in 3 mL of LB medium including 50 µg/mL kanamycin at 37°C for 5 to 7 hours at 250 rpm, and then, 1 mL was transferred to 10 mL, diluted 10-fold and further cultured. After about 2 to 4 hours, when O.D600 reached 0.5 to 0.7, 0.4 mM IPTG (Biosesang, Cat#I1006, Lot#LB0C0340, cas#367-93-1) was added, and it was cultured at 16°C for 17 to 19 hours (maximum 21 hours). The culture solution was centrifuged to obtain only the precipitated *E. coli* and stored in an ultra-low temperature freezer at -80°C. 0.3 mL of lysis buffer (50 mM Tris-HCl (pH 7.5), 300 mM NaCl, 5% glycerol, 0.1% β-Mercaptoethanol and 10 mM imidazole) was added to the *E. coli* harvest corresponding to 3 mL of culture medium, and it was sonicated. Afterwards, the total lysate (T) was separated into a precipitant (P) and a soluble lysate (S) through centrifugation, and as a result of analysis by SDS-PAGE, as shown in FIG. 1d, in the case of the norovirus GII.17 VP1 protein with RID added to the N-terminus, it was confirmed that the expression was highly efficient but mostly insoluble, whereas the norovirus GII.17 VP1 protein with the mutant RID added to the N-terminus showed a highly efficient soluble expression.

### <Example 1-3>

Using the same culture method as Example 1-2 in which high-efficiency soluble expression was confirmed, 500 mL of *E. coli* was cultured, and expression was induced, and the obtained cells were resuspended in 75 mL of lysis buffer solution A buffer [50 mM Tris-HCl (pH 7.5), 300 mM NaCl, 5% glycerol, 0.1% β-Mercaptoethanol and 10 mM imidazole]. The cell resuspension product was disrupted by using an ultrasonic cell disruptor. The dissolved cells were centrifuged at 13,500 rpm at 4°C for 10 minutes, and the soluble lysate was collected and subjected to Ni+ affinity chromatography using AKTA (GE Healthcare). First of all, a Ni Sepharose High Performance (Cytiva) column was equilibrated with buffer A (50 mM Tris-HCl (pH 7.5), 300 mM NaCl, 5% glycerol, 0.1% β-Mercaptoethanol and 10 mM imidazole), and then, the cell soluble lysate was loaded onto the equilibrated column at a flow rate of 3 to 5 mL/minutes. Norovirus GII.17 VP1 protein was eluted and obtained by increasing the imidazole concentration in a linear gradient from 10 mM to 300 mM using buffer A and buffer B including 300 mM imidazole [50 mM Tris-HCl (pH 7.5), 300 mM NaCl, 5% glycerol, 0.1% β-Mercaptoethanol and 10 mM imidazole].

In order to determine the separation pattern of the target protein through SDS-PAGE of the above extract, the gel was analyzed by using the Image Lab (Ver. 6.1) program. After designating the Total Lysate Band as a Reference Band using the Quantity Tool in the program, the soluble lysate Relative Quantity value was obtained to compare and analyze the degree of soluble (or water-soluble) protein expression of each construct. As a result, as shown in FIG. 1c, it was confirmed that the soluble protein expression rate and the protein yield after purification of the fusion protein including m3c among the RIDs with three mutations were significantly higher than those including m3a.

### <Example 1-4>

### Confirmation of VLP formation according to the type of norovirus antigen protein binding fusion protein

The appearance of virus-like particles (VLPs) formed by the purified norovirus GII.17 VP1 protein was observed by using an electron microscope. The VLP composed of norovirus VP1 was first placed on a copper grid for 1 minute, then stained with 2% uranyl acetate for 1 minute, dried at room temperature for 10 minutes, and then purified by using a transmission electron microscope (TEM; Talos L120C, FEI, Czech) to confirm the formation of VLPs of the assembled norovirus VP1 protein. As shown in FIG. 1e, the diameter of the confirmed VLPs was spherical VLPs with a diameter of 30 to 40 nm, which is the diameter of norovirus particles. In particular, it was confirmed that the norovirus GII.17 VP1 protein fused with the mutant RID including m3c formed homogeneous VLPs having almost similar sizes with high efficiency. On the other hand, in the case of other mutant RIDs, VLP formation was observed, but the efficiency was low, and it was confirmed that many intermediates and impurities were included.

Through the results of Example 1 above, when the norovirus VP1 antigen protein including the mutant RID with the RNA binding site preserved according to the present invention was expressed, it was confirmed that not only was a much higher level of expression achieved, but also that proper protein folding was induced, thereby showing higher solubility, and the efficiency and homogeneity of VLP assembly by the final purified VP1 protein were excellent.

### [Example 2]

### Preparation of norovirus GII.17 VP1 virus-like particles (VLPs) using an expression level and solubility enhancing partner (RID) at the N-terminus

### <Example 2-1>

The two expression vectors of Example 1-1 above (RID-NoV GII.17 VP1 expression vector, RID (EE domain + m3c)-NoV GII.17 VP1 expression vector) were transformed into HMS174 competent cells (Novagen (RecA mutation in a K-12 strain, Cat: 69453-3)). All transformed *E. coli* were cultured in 3 mL of LB medium including 50 µg/ml kanamycin at 37°C and 250 rpm for 5 to 7 hours, and 1 mL was transferred to 10 mL, diluted 10-fold and further cultured. After about 2 to 4 hours, when the O.D600 reached 0.5 to 0.7, 0.4 mM IPTG (Biosesang, Cat#I1006, Lot#LB0C0340, cas#367-93-1) was added, and it was cultured at 16°C for 17 to 19 hours (maximum 21 hours). The culture solution was centrifuged to obtain only the precipitated *E*. *coli* and stored in an ultra-low temperature freeze at -80°C. 0.3 mL of lysis buffer (50 mM Tris-HCl (pH 7.5), 300 mM NaCl, 5% glycerol, 0.1% β-Mercaptoethanol and 10 mM imidazole) was added to the *E. coli* harvest corresponding to 3 mL of culture medium, and it was sonicated. Afterwards the total lysate (T) was separated into the precipitant (P) and the soluble lysate (S) by centrifugation, and as a result of analysis by SDS-PAGE, it was confirmed that the norovirus GII.17 VP1 protein with the mutant RID added to the N-terminus exhibited highly efficient soluble expression, as shown in FIG. 2a.

### <Example 2-2>

Using the same culture method as Example 1-2 in which high-efficiency soluble expression was confirmed, 500 mL of *E. coli* was cultured and expression was induced, and the obtained cells were resuspended in 75 mL of lysis buffer solution A buffer [50 mM Tris-HCl (pH 7.5), 300 mM NaCl, 5% glycerol, 0.1% β-Mercaptoethanol, and 10 mM imidazole]. The cell resuspended product was disrupted by using an ultrasonic cell disruptor. The dissolved cells were centrifuged at 13,500 rpm at 4°C for 10 minutes, and the soluble lysate was collected and subjected to Ni+ affinity chromatography using AKTA (GE Healthcare). First of all, a Ni Sepharose High Performance (Cytiva) column was equilibrated with buffer A (50 mM Tris-HCl (pH 7.5), 300 mM NaCl, 5% glycerol, 0.1% β-Mercaptoethanol and 10 mM imidazole), and then, the cell soluble lysate was loaded onto the equilibrated column at a flow rate of 3 to 5 mL/minutes. Norovirus GII.17 VP1 protein was eluted and obtained by increasing the imidazole concentration in a linear gradient from 10 mM to 300 mM using buffer A and buffer B including 300 mM imidazole [50 mM Tris-HCl (pH 7.5), 300 mM NaCl, 5% glycerol, 0.1% β-Mercaptoethanol and 10 mM imidazole].

After determining the separation pattern of the target protein through SDS-PAGE of the above eluate, the eluate of the corresponding fraction was dialyzed in Dialysis buffer [50 mM Tris-HCl (pH 8.5), 10 mM NaCl, 5% glycerol, 0.1% β-Mercaptoethanol] at 4°C overnight (12 to 16 hours) to cleave between the N-terminal RID and the norovirus antigen protein by using TEV cleavage enzyme.

In order to isolate and purify the target protein from which the N-terminal fusion partner RID and the like were removed, a Ni Sepharose High Performance (Cytiva) column was equilibrated with the same solution A buffer as the dialysis buffer [50 mM Tris-HCl (pH 8.5), 10 mM NaCl, 5% glycerol, 0.1% 2-Mercaptoethanol], and then loaded onto the equilibrated column at a flow rate of 3 to 5 mL/minutes. The NoV GII.17 target protein from which the N-terminal fusion partner RID and the like were removed was recovered in the form of being eluted without being adsorbed to the column.

In order to increase the final purity of the target protein, ion exchange resin chromatography was performed by using AKTA (GE healthcare). The column (Fractogel EMD DEAE(M), Merck Millipore) was equilibrated with buffer solution A buffer [50 mM Tris-HCl (pH 8.5), 10 mM NaCl, 5% glycerol, 0.1% β-Mercaptoethanol] and loaded at a flow rate of 1 ml/minutes. The target protein was eluted through a linear concentration gradient of 10 mM to 1 M NaCl injection with buffer solution AA and buffer solution BB [50 mM Tris-HCl (pH 8.5), 1 M NaCl, 5% glycerol, 0.1% β-Mercaptoethanol], and then obtained as fractions of 1 ml each. As a result, the purification pattern and purity were as described in FIGS. 2b, 2c and 2d (refer to FIG. 2). The concentration of the finally purified protein was quantified by using BSA (Amresco, Solon, OH, USA). Referring to FIG. 2, it can be seen that the RID protein bound to the fusion protein was separated, indicating that the purification of the target protein was successful.

### <Example 2-3>

The norovirus VP1 protein purified according to Example 2-2 was stored at 4°C after exchanging the buffer overnight (12 to 16 hours) to the final state of Assembly buffer [100 mM potassium phosphate pH 5.8, 150 mM NaCl, 10% glycerol] including 10% glycerol, and the purified norovirus VP1 protein was assembled into a VLP form through a series of processes.

### <Example 2-4>

In order to determine the overall diameter distribution of the virus-like particles (VLPs) assembled with the purified norovirus VP1 protein according to Example 2-3, it was analyzed through dynamic light scattering (DLS). The analysis of the complex was measured by placing 1 mL of the sample in a cuvette at a temperature of 16°C using DLS (Particular Systems, Zetasizer Nano Family). The dynamic light scattering analysis of the cell was confirmed by using the total intensity and mass of each area in the image. As shown in FIG. 2e, it was confirmed that particles were formed with a size of 30 to 40 nm in diameter at the hydrodynamic radius of DLS (DLS values may have interference depending on the wavelength of light).

### <Example 2-5>

The appearance of the virus-like particles (VLPs) formed by the purified norovirus GII.17 protein was observed by using an electron microscope. The purified VLPs were first placed on a copper grid for 1 minute, then stained with 2% uranyl acetate for 1 minute, dried at room temperature for 10 minutes, and photographed by using a transmission electron microscope (TEM, 120kV; Talos L120C, FEI, Czech). As shown in FIG. 2f, it was confirmed that the purified and assembled norovirus VP1 protein formed VLP. The diameter of the confirmed VLP was confirmed to be between 30 and 40 nm, which is the diameter of the norovirus particle, and it was confirmed that highly homogeneous spherical VLPs were formed (refer to FIG. 2f).

### <Example 2-6>

In order to measure the degree to which the purified GII.4, GII.17 and GII.3 VLPs according to the present invention bind to HBGA, an HBGA binding assay was performed. As shown in FIG. 2g, 5 types of biotin-tagged HBGA (H (type 2)-PAA-biotin (01-034), Lea-PAA-biotin (01-035), Led (H type1)-PAA-biotin (01-037), Blood type A (tri)-PAA-biotin (01-032), Blood type B(tri)-PAA-biotin(01-033);(Glycotech)) were added 100 µL per well at a concentration of 10 µg/mL to streptavidin-coated 96-well plates (High Binding Capacity (HBC) NeutrAvidin plates (Thermo Scientific^{™})) and reacted at room temperature for 5 hours. After washing the plate with PBST, GII.17 VLP protein was diluted in PBS, added 100 µL per well, and reacted at 4°C for 18 hours. After washing the plate with PBST, rabbit NoV GII.17 polyclonal IgG Ab was diluted 2,000-fold with PBS, 100 µL per well was added, and it was reacted at room temperature for 1 hour and 30 minutes. After washing with PBST, goat-derived rabbit IgG (Goat-anti rabbit IgG HRP) was diluted 10,000-fold with PBS, 100 µL per well was added, and it was treated at room temperature for 1 hour and 30 minutes. Thereafter, after color development for 5 minutes at room temperature using TMB solution, the color development reaction was stopped with 0.5 M sulfuric acid solution, and the absorbance was measured at a wavelength of 450 nm. The binding between norovirus VLPs and five HBGAs was confirmed, and as a result, GII.4, GII.17 and GII.3 VLP proteins were confirmed to strongly bind to Blood type B (tri), Blood type B (tri) and Le a (H type1), respectively, and the degree of binding was expressed as EC50, as shown in FIG. 2h.

### [Example 3]

### Confirmation of immunogenicity of GII.17 VLP formed using mRID

### <Example 3-1>

In order to measure the ability to induce norovirus GII.17 antigen-specific antibodies, 500 µL of virus-like particles prepared according to the present invention (VLPs, VLPs composed of purified antigen proteins after RID is cleaved from the fusion protein of the present invention (RID (EE domain + m3c) - NoV GII.17 VP1)) were inoculated 500 µL through intramuscular injection into a group of 7-week-old SD (Sprague Dawley) rats (5 mice/group) according to concentration (FIG. 3a). A syringe equipped with a 26-gauge needle was used to administer a predetermined amount of the dose (500 µL/head) into both thighs of the animals, and 0.5 mg of norovirus G II.17 VLP antigen and an immune booster, alum hydroxide, were mixed. The vaccine was inoculated three times in total at two-week intervals, and about 1 mL of whole blood was collected through the jugular vein on a scheduled date and injected into a vacutainer tube containing a clot activator.

An enzyme-linked immunosorbent assay (ELISA) was performed to measure the target protein-specific antibody titer of the serum obtained from each animal experimental group. 100 µL of norovirus GII.17 VLP was added to each well of a 96-well plate (High Binding Capacity (HBC) NeutrAvidin plates (Thermo Scientific^{™})) at a concentration of 0.5 µg/mL and coated overnight at 4°C. T hen, 200 µL of 3% BSA solution was added and blocked by reaction at 37°C for 2 hours. After washing the plate with PBST, 100 µL of immune serum was diluted (1:1,600 dilution) and added to each well, and it was reacted in the same manner at 37°C for 1 hour and 30 minutes. After washing the plate with PBST, the serum was treated with an antibody against goat-derived mouse IgG (southern Biotech 1030-05, Goat-anti mouse IgG HRP) at 37°C for 1 hour to detect antibodies bound to GII.17 VLP. Thereafter, after 5 minutes of color development using TMB (sigma T0440) solution at room temperature, the color development reaction was stopped with a 0.5 M sulfuric acid solution, and the absorbance was measured at a wavelength of 450 nm. As a result of confirming the GII.17 VLP-specific antibody titer in the blood samples after inoculation at 8,000-fold dilution from the test groups 1 µg/head, 5 µg/head, 10 µg/head and 20 µg/head, as shown in FIGS. 3ba to 3bc, it was confirmed that significant GII.17 VLP antigen-specific antibodies were formed at all three concentrations of 5 ug, 10 ug and 20 ug with only the second inoculation. In detail, in the 0.2 µg/head test group, antibody production was confirmed in all subjects from 14 days after the second administration.

### <Example 3-2>

In order to measure the degree to which immune serum inhibits the binding between GII.17 VLP antigen (VLP composed of purified antigen protein after RID (EE domain + m3c)-NoV GII.17 VP1 fusion protein of the present invention is cleaved, Nov VLP in FIG. 3c) and HBGA, an HBGA blocking assay was performed on immune serum obtained according to the schedule after inoculation (FIG. 3c). Blood type B(tri)-PAA-biotin(01-033);(Glycotech), which has high binding affinity for GII.17 type, was added at a concentration of 10 µg/mL (100 µL per well) to a 96-well plate (High Binding Capacity (HBC) NeutrAvidin plates (Thermo Scientific^{™})) coated with streptavidin, and it was reacted for 5 hours at room temperature. The primary, secondary and tertiary immune sera to determine the inhibitory efficacy of antigen and HBGA binding were serially diluted from 100-fold to 2-fold in PBS and reacted with GII.17 VLP protein diluted to an appropriate concentration at 37°C for 1 hour and 30 minutes. 100 µL per well of the serum and GII.17 VLP reaction solution were added to the plate reacted with HBGA and reacted at 4°C for 18 hours. After washing the plate with PBST, rabbit NoV GII.17 polyclonal IgG Ab was diluted 2,000-fold with PBS, 100 µL per well was added, and the plate was reacted at room temperature for 1 hour and 30 minutes. After washing with PBST, goat-derived rabbit IgG (Goat-anti rabbit IgG HRP) was diluted 30,000-fold with PBS, 100 µL per well was added, and the plate was treated at room temperature for 1 hour and 30 minutes. Thereafter, after 5 minutes of the color development using TMB solution at room temperature, the color development reaction was stopped with 0.5 M sulfuric acid solution, and the absorbance was measured at a wavelength of 450 nm.

FIG. 3d is the results of the HBGA binding inhibition test in immune serum (using B tri synthetic carbohydrate), confirming the HBGA inhibition efficacy from 5 µg/head (low concentration), 10 µg/head (medium concentration) and 20 µg/head (high concentration) 14 days after the first, second and third inoculations, respectively. According to FIG. 3d, the HBGA binding inhibition efficacy increased dose-dependently from the first immune serum, and when the GII.17 VLP results were analyzed, the HBGA binding inhibition efficacy of 1:200 or more was confirmed at all administered doses after the first inoculation, the high HBGA binding inhibition efficacy of 1:5,000 or more was confirmed at all doses after the second inoculation, and the high HBGA binding inhibition efficacy of 1:6,000 or more was confirmed at all doses after the third inoculation. The degree of inhibition of VLP antigen-HBGA binding was not significant at all concentrations, but in the second and third immune sera, the degree of inhibition of VLP antigen-HBGA binding was higher at all concentrations compared to after the first inoculation, and the difference in inhibition efficacy by administered concentration between the low-concentration group and the medium and high-concentration groups was large and saturated.

Referring to the results of Example 3 above, when the norovirus GII.17 antigen protein was expressed in the form of a fusion protein with the existing RID, it maintained activity as a fusion partner that assists the folding of the target protein such that the soluble expression level was excellent, and the protein structure was stable by overcoming protein misfolding, and the effect of improving self-folding and assembly ability was confirmed. Through this, it was confirmed that not only were the efficiency and homogeneity of VLP assembly by the finally purified norovirus GII.17 VP1 protein excellent, but also the antibody induced through the inoculation of the VLPs had an excellent performance in inhibiting binding to HBGA, which plays a key role in intracellular infection of norovirus.

### [Example 4]

Preparation of norovirus GII.3 VP1 virus-like particles (VLPs) added with expression level and solubility-enhancing partners of N-terminus for high efficiency and solubility enhancement

### <Example 4-1>

In order to produce virus-like particles composed of norovirus GII.3 VP1 protein, the VP1 gene derived from norovirus GII.3 protein (Norovirus Hu/Gll.3/CHDC2005/1975/US, NCBI access number: HM072045.1) was used. VP1, which is an antigen of norovirus, consists of 547 amino acids, and the molecular weight of the protein is approximately 59.7 kDa. Based on the protein sequence, a gene sequence synthesized after codon optimization in *E*. *coli* was prepared (SEQ ID NO: 10) (Request for gene synthesis to Bionics Co., Ltd.).

In FIG. 4a, the EE domain (MSAVKAA (SEQ ID NO. 2)) with the effect of increasing expression level and increasing solubility at the N-terminus of the protein and the SE domain (SEQ ID NO. 3) or a mutant SE domain peptide (SEQ ID NO. 7) which is more than 50% similar to SEQ ID NO.3 and includes mutations of K23A, K30A, and K31A were linked to prepare a recombinant fusion protein, and the solubility expression pattern was verified.

Referring to FIG. 4a, in the case of the fusion protein (RID (EE domain + m3c) - NoV GII.3 VP1) in which RID (EE domain + m3c) - (mutant RID peptide including mutations of K23A, K30A, and K31A) was linked, it can be confirmed that the solubility (s) expression pattern was much higher than that of the fusion protein (RID-NoV GII.3 VP1) in which RID without mutation was linked.

In addition, a peptide having an amino acid sequence of (GaSb)ₙ (a≥1, b≥1, n≥1) was used as a linker of an optimized sequence by applying the sequence of the TEV cleavage site, ENLYFQG/S, and this was inserted between the TEV cleavage sequence (ENLYFQ) and the target protein (norovirus antigen protein) sequence. A mimetic diagram of the fusion protein expression vector prepared in this example is as shown in FIG. 1a.

The gene of the above sequence was prepared based on the recombinant complex protein expression vectors, pET-9a-EE domain(MSAVKAA)-SE domain(wt)-Nov GII.17 VP1 and pET-9a- EE domain(MSAVKAA)-m3c-Nov GII.17 VP1 vectors prepared in Example 1-1 above by using the pET-9a (Novagene, 69431-3CN) (SEQ ID NO: 1) expression vector. T7 RNA polymerase was expressed by IPTG, and through this, the Lac operon existing on the DE3 genome and the MCS (Multiple Cloning Site) of the pET vector, where the T7 promoter operates, were inserted into the existing N-terminus, and the RID sequence for increasing solubility and expression rate, and the C-direction of the linking site sequence to enhance 6xHIS and TEV cleavage efficiency were reinserted by using KpnI and BamHI cleavage enzymes. Among the cleavage enzyme sites within the MCS, the RID, cleavage enzyme site and the linker site for increasing the cleavage efficiency of the TEV enzyme were inserted between the Ndel and Kpnl sequences, and the DNA sequences designed to have a protein in the form of a fusion of the norovirus VP1 antigen protein were inserted between the Kpnl and BamHI cleavage enzymes, and each gene was linked to the pET vector and the inserted gene sequence through T4 DNA ligase. The bacterial clones generated by the DNA ligation reaction were confirmed to have the sequence of the codon-optimized nucleotide inserted through DNA sequence analysis.

The sequences of the recombinant complex expression vectors prepared in Example 4-1 are as follows:
EE domain (MSAVKAA)-SE domain-GT-6xHIS-TEV-GS-NoV GII.3 VP1 [(SEQ ID NO. 2)-(SEQ ID NO. 3)-GT-6xHIS-TEV-GS-(SEQ ID NO. 10)]:

MSAVKAA-
AAVQAAEVKVDGSEPKLSKNELKRRLKAEKKVAEKEAKQKELSEKQLSQATAAATNHTTDNGV
GPEEESV-GT-HHHHHH-ENLYFQ-GS-
KMASNDAAPSNDGAAGLVPEINNEAMALEPVAGAAIAAPLTGQQNIIDPWIMNNFVQAPG
GEFTVSPRNSPGEVLLNLELGPEINPYLAHLARMYNGYAGGFEVQVVLAGNAFTAGKVIFAAIP
PNFPIDNLSAAQITMCPHVIVDVRQLEPINLPMPDVRNNFFHYNQGSDSRLRLIAMLYTPLRA
NNSGDDVFTVSCRVLTRPSPDFSFNFLVPPTVESKTKPFTLPILTISEMSNSRFPVPIDSLHTSPTE
NIVVQCQNGRVTLDGELMGTTQLLPSQICAFRGTLTRSTSRAGDQADTATPRLFNYYWHIQLD
NLNGTPYDPAEDIPAPLGTPDFRGKVFGVASQRNPDSTTRAHEAKVDTTSGRFAPKLGSLEIST
ESSDFDSNQPTRFTPVGIGVDNEADFQQWSLPDYSGQFTHNMNLAPAVAPNFPGEQLLFFRS
QLPSSGGRSNGILDCLVPQEWVQHFYQESAPAQTQVALVRYVNPDTGRVLFEAKLHKLGFMT
IAKNGDSPITVPPNGYFRFESWVNPFYTLAPMGTGNGRRRIQ

The prepared complex above was named as "RID-NoV GII.3 VP1."

MSAVKAA- m3c-GT-6xHIS-TEV-GS-NoV GII.3 VP1 [(SEQ ID NO. 2)-(SEQ ID NO. 7)-GT-6xHIS-TEV-GS-(SEQ ID NO. 10)]

:MSAVKAA-
AAVQAAEVKVDGSEPKLSKNELARRLKAEAAVAEKEAKQKELSEKQLSQATAAATNHTTDNGV
GPEEESV
-GT-HHHHHH-ENLYFQ-GS-
KMASNDAAPSNDGAAGLVPEINNEAMALEPVAGAAIAAPLTGQQNIIDPWIMNNFVQAPG
GEFTVSPRNSPGEVLLNLELGPEINPYLAHLARMYNGYAGGFEVQVVLAGNAFTAGKVIFAAIP
PNFPIDNLSAAQITMCPHVIVDVRQLEPINLPMPDVRNNFFHYNQGSDSRLRLIAMLYTPLRA
NNSGDDVFTVSCRVLTRPSPDFSFNFLVPPTVESKTKPFTLPILTISEMSNSRFPVPIDSLHTSPTE
NIVVQCQNGRVTLDGELMGTTQLLPSQICAFRGTLTRSTSRAGDQADTATPRLFNYYWHIQLD

NLNGTPYDPAEDIPAPLGTPDFRGKVFGVASQRNPDSTTRAHEAKVDTTSGRFAPKLGSLEIST
ESSDFDSNQPTRFTPVGIGVDNEADFQQWSLPDYSGQFTHNMNLAPAVAPNFPGEQLLFFRS
QLPSSGGRSNGILDCLVPQEWVQHFYQESAPAQTQVALVRYVNPDTGRVLFEAKLHKLGFMT
IAKNGDSPITVPPNGYFRFESWVNPFYTLAPMGTGNGRRRIQ

The prepared complex above was named as "RID(EE domain+m3c)-NoV GII.3 VP1."

### <Example 4-2>

The two expression vectors of Example 4-1 above were transformed into HMS174 competent cells (Novagen (RecA mutation in a K-12 strain, Cat: 69453-3)). All transformed *E. coli* were cultured in 3 mL of LB medium including 50 µg/mL kanamycin at 37°C for 5 to 7 hours at 250 rpm, and then, 1 mL was transferred to 10 ml, diluted 10-fold and further cultured. After about 2 to 4 hours, when O.D600 reached 0.5 to 0.7, 0.4 mM IPTG (Biosesang, Cat#I1006, Lot#LB0C0340, cas#367-93-1) was added and cultured at 16°C for 17 to 19 hours (maximum 21 hours). The culture solution was centrifuged to obtain only the precipitated *E. coli* and stored in an ultra-low temperature freezer at -80°C. 0.3 mL of lysis buffer (50 mM Tris-HCl (pH 7.5), 300 mM NaCl, 5% glycerol, 0.1% β-Mercaptoethanol, and 10 mM imidazole) was added to the *E. coli* harvest corresponding to 3 mL of culture medium, and it was sonicated. After the total lysate (T) was separated into the precipitant (P) and the soluble lysate (S) through centrifugation, as a result of analysis by SDS-PAGE, it was confirmed that as shown in FIG. 4a, the norovirus GII.3 VP1 protein (RID (EE domain + m3c) - NoV GII.3 VP1 fusion protein; right side in FIG. 4a) with a mutant RID containing an RNA binding site added to the N-terminus exhibited a highly efficient soluble expression.

### <Example 4-3>

In order to obtain the RID(EE domain+m3c)-NoV GII.3 VP1 fusion protein, 500 mL of *E. coli* was cultured and expression was induced by using the same culture method as in Example 1-2, which confirmed high-efficiency soluble expression, and the obtained cells were resuspended in 75 mL of lysis buffer solution A buffer [50 mM Tris-HCl (pH 7.5), 300 mM NaCl, 5% glycerol, 0.1% β-Mercaptoethanol and 10 mM imidazole]. The cell resuspended product was disrupted by using an ultrasonic cell disruptor. The dissolved cells were centrifuged at 13,500 rpm at 4°C for 10 minutes, and the soluble lysate was collected and subjected to Ni+ affinity chromatography using AKTA (GE Healthcare). First of all, a Ni Sepharose High Performance (Cytiva) column was equilibrated with buffer A (50 mM Tris-HCl (pH 7.5), 300 mM NaCl, 5% glycerol, 0.1% β-Mercaptoethanol and 10 mM imidazole), and then, the cell soluble lysate was loaded onto the equilibrated column at a flow rate of 3 to 5 mL/minutes. Norovirus GII.3 VP1 protein was eluted and obtained by increasing the imidazole concentration in a linear gradient from 10 mM to 300 mM using buffer A and buffer B including 300 mM imidazole [50 mM Tris-HCl (pH 7.5), 300 mM NaCl, 5% glycerol, 0.1% β-Mercaptoethanol and 10 mM imidazole].

After determining the separation pattern of the target protein through SDS-PAGE of the above eluate, the eluate of the corresponding fraction was dialyzed in Dialysis buffer [50 mM Tris-HCl (pH 8.5), 10 mM NaCl, 5% glycerol, 0.1% β-Mercaptoethanol] at 4°C overnight (12to 16 hours), and the N-terminal RID and the norovirus antigen protein VP1 were cleaved by using TEV cleavage enzyme.

In order to isolate and purify the target protein from which the N-terminal fusion partner RID and the like were removed, a Ni Sepharose High Performance (Cytiva) column was equilibrated with the same solution A buffer as the dialysis buffer [50 mM Tris-HCl (pH 8.5), 10 mM NaCl, 5% glycerol, 0.1% 2-Mercaptoethanol], and then loaded onto the equilibrated column at a flow rate of 3 to 5 mL/minutes. The NoV GII.-3 target protein from which the N-terminal fusion partner RID and the like were removed was recovered in the form of being eluted without being adsorbed to the column.

In order to increase the final purity of the target protein, ion exchange resin chromatography was performed using AKTA (GE Healthcare). The column (Fractogel EMD DEAE(M), Merck Millipore) was equilibrated with buffer solution A buffer [50 mM Tris-HCl (pH 8.5), 10 mM NaCl, 5% glycerol, 0.1% β-Mercaptoethanol] and loaded at a flow rate of 1 mL/minute. The target protein was eluted through the injection of NaCl having a linear concentration gradient of 10 mM to 1 M with buffer solution AA and buffer solution BB [50 mM Tris-HCl (pH 8.5), 1 M NaCl, 5% glycerol, 0.1% β-Mercaptoethanol] and obtained as fractions of 1 mL each. As a result, the purification pattern and purity thereof are as described in FIGS. 4b, 4c and 4d. The concentration of the finally purified protein was quantified by using BSA (Amresco, Solon, OH, USA). Referring to FIGS. 4b to 4d, it can be seen that the RID protein bound to the fusion protein was separated, indicating that the purification of the target protein was successful.

### <Example 4-4>

The purified norovirus VP1 protein was stored at 4°C after exchanging the buffer overnight (12 to 16 hours) to the final state of Assembly buffer [100 mM potassium phosphate pH 5.8, 150 mM NaCl, 10% glycerol] including a 10% glycerol ratio, and the concentration of the final purified protein was quantified by using BSA (Amresco, Solon, OH, USA).

### <Example 4-5>

In Example 4-4, the fusion protein (Norovirus VP1 fusion protein including RID mutant protein (RID (EE domain + m3c) - NoVGII.3 VP1 fusion protein)) was purified after RID cleavage to determine the overall diameter distribution of the self-assembled virus-like particles (VLPs) by using dynamic light scattering (DLS). The analysis of the complex was measured by placing 1 mL of the sample in a cuvette at a temperature of 16°C using DLS (Particular Systems, Zetasizer Nano Family). The dynamic light scattering analysis of the cell was confirmed by using the total intensity and mass of each area in the image. As shown in FIG. 4e, it was confirmed that the particles were formed with a size distribution of 30 to 40 nm in diameter at the hydrodynamic radius of DLS.

### <Example 4-6>

According to Example 4-4, the appearance of virus-like particles (VLPs) assembled with the target protein purified from the fusion protein (Norovirus VP1 fusion protein (RID (EE domain + m3c) - NoV GII.3 VP1 fusion protein including RID mutant protein) was observed by using an electron microscope. The purified norovirus VLPs were first placed on a copper grid for 1 minute, then stained with 2% uranyl acetate for 1 minute, dried at room temperature for 10 minutes, and then photographed by using a transmission electron microscope (TEM, 120 kV; Talos L120C, FEI, Czech). As shown in FIG. 4f, it was confirmed that the purified and assembled norovirus VP1 protein formed VLPs. The diameter of the confirmed virus-like particles (VLPs) appeared to be between 30 and 40 nm, which is the diameter of the norovirus particles, and it was confirmed that spherical VLPs with high homogeneity were formed.

In the results of Example 4, as in the results of Example 2, it was confirmed that when the mutant RID according to the present invention and the norovirus antigen protein were fused, much higher efficiency of target protein expression, proper folding induction, and VLP assembly ability were improved in *E. coli.*

### [Example 5]

### Confirmation of immunogenicity of GII.3 VLP formed by using mRID

### <Example 5-1>

In order to measure the norovirus GII.3 antigen-specific antibody induction ability, 500 µL of virus-like particles (VLPs, VLPs composed of purified antigen protein after RID is cleaved from the fusion protein of the present invention (RID (EE domain + m3c) - NoV GII.3 VP1)) prepared according to the present invention were inoculated through intramuscular injection into 7-week-old SD (Sprague Dawley) rats (5 mice/group) according to the concentration (FIG. 5a). Using a syringe equipped with a 26-gauge syringe needle, a predetermined amount of the solution (500 µL/head) was divided and administered into the thighs of both sides of the animals, and 0.5 mg of norovirus G II.3 VLP antigen and the immune booster, alum hydroxide, were mixed. The vaccine was inoculated and immunized three times in total at two-week intervals, and about 1 mL of whole blood was collected through the jugular vein at the scheduled time and injected into a vacutainer tube containing a clot activator.

An enzyme-linked immunosorbent assay (ELISA) was performed to measure the target protein-specific antibody titer of the serum obtained from each animal experimental group. 100 µL of norovirus GII.3 VLP was added to each well of a 96-well plate (High Binding Capacity (HBC) NeutrAvidin plates (Thermo Scientific^{™})) at a concentration of 0.5 µg/mL and coated overnight at 4°C. Then, 200 µL of 3% BSA solution was added and blocked by reaction at 37°C for 2 hours. After washing the plate with PBST, 100 µL of immune serum was diluted (1:1,600 dilution) and added to each well and reacted in the same manner at 37°C for 1 hour and 30 minutes. After washing the plate with PBST, the serum was treated with an antibody against goat-derived mouse IgG (southern Biotech 1030-05, Goat-anti mouse IgG HRP) at 37°C for 1 hour to detect antibodies bound to GII.3 VLP. Afterwards, the plate was incubated with TMB (sigma T0440) solution for 5 minutes at room temperature, the color development reaction was stopped with a 0.5 M sulfuric acid solution, and the absorbance was measured at a wavelength of 450 nm. As shown in FIG. 5ba, the GII.3 VLP-specific antibody titer in blood samples collected after inoculation was confirmed at 8,000-fold dilution from the test groups of 0.2 µg/head, 1 µg/head, 5 µg/head, 10 µg/head and 20 µg/head. As a result, the specific IgG antibody titer was confirmed in the group administered 1 µg or more after the first inoculation, and the production of high specific IgG antibody titers was confirmed in all concentration groups after the second inoculation (FIGS. 5bb, 5bc).

### <Example 5-2>

In order to measure the degree to which immune serum inhibits the binding between GII.3 VLP antigen (VLPs composed of purified antigen protein after RID (EE domain + m3c) - NoV GII.3 VP1 fusion protein of the present invention is cleaved, Nov GII.3 VLP in FIG. 5c) and HBGA, an HBGA blocking assay was performed on immune serum obtained according to the schedule after inoculation (FIG. 5c). 100 µL of Lea-PAA-biotin (01-035); (Glycotech), which has high binding affinity for GII.3 type, was added to each well at a concentration of 10 µg/mL in a streptavidin-coated 96-well plate (High Binding Capacity (HBC) NeutrAvidin plates (Thermo Scientific^{™})) and reacted at room temperature for 5 hours. In order to determine the inhibitory efficacy of VLP antigen binding to HBGA, the first, second and third immune sera were serially diluted 100-fold to 2-fold in PBS, respectively, and reacted with GII.3 VLP protein diluted to an appropriate concentration at 37°C for 1 hour and 30 minutes. The serum and GII.3 VLP reaction solution were added 100 µL per well to the plate reacted with HBGA, and it was reacted at 4°C for 18 hours. After washing the plate with PBST, rabbit NoV GII.3 Polyclonal Ig G Ab was diluted 2,000-fold in PBS, added 100 µL per well, and reacted at room temperature for 1 hour and 30 minutes. After washing with PBST, goat-anti rabbit IgG HRP was diluted 30,000-fold in PBS, added 100 µL per well, and treated at room temperature for 1 hour and 30 minutes. Thereafter, after 5 minutes of the color development at room temperature using TMB solution, the color development reaction was stopped with 0.5 M sulfuric acid solution, and the absorbance was measured at a wavelength of 450 nm.

FIG. 5c shows the results of the HBGA binding inhibition test in immune serum (using Le a synthetic carbohydrate), and the HBGA inhibition efficacy was confirmed from 5 µg/head (low concentration), 10 µg/head (medium concentration) and 20 µg/head (high concentration) on day 14 after the first, second and third inoculations, respectively. As a result of the GII.3 analysis, the HBGA binding inhibition efficacy of 1:200 or more was confirmed at all doses after the first inoculation, and after the second inoculation, a high HBGA binding inhibition efficacy of 1:1,500 or more, which was significantly increased compared to the first administration, was confirmed at all doses, and after the third inoculation, a high HBGA binding inhibition efficacy of 1:2,500 or more was confirmed at all doses.

After the first administration, similar HBGA binding inhibition effects were observed in all dose groups, and after the second administration, much higher efficacy was induced compared to the first inoculation, and higher HBGA binding inhibition efficacy was induced in the 10 µg and 20 µg administration groups compared to the 5 µg administration group. After the third inoculation, even higher HBGA binding inhibition efficacy was induced, and regardless of the administration dose, an overall saturation pattern was observed, confirming that the antigen exhibited high immunogenicity.

In the results of Example 5, as in the results of Example 3, when the norovirus GII.3 antigen protein according to the present invention was expressed in the form of a fusion protein with an existing RID, it maintained activity as a fusion partner that assisted the folding of the target protein such that the soluble expression level was excellent, the protein structure was stable by overcoming protein misfolding, and the effect of improving self-folding and assembly ability was confirmed. Through this, it was confirmed that not only were the efficiency and homogeneity of VLP assembly by the finally purified norovirus GII.3 VP1 protein excellent, but also the antibody induced through the inoculation of the corresponding VLPs had an excellent performance in inhibiting binding to HBGA, which plays a key role in the intracellular infection of norovirus.

### [Example 6]

### Preparation of VP1 virus-like particles (VLPs) according to the genotype of the norovirus antigen target protein purified from the fusion protein

In order to produce virus-like particles composed of the target protein (norovirus antigen protein) purified from the fusion protein of the norovirus VP1 protein to which the RID sequence for high-efficiency soluble expression was attached at the N-terminus, the gene of VP1 derived from the proteins of each genotype GI.1 (Norovirus Hu/GI.1/8McIII/1973/USA, NCBI access number: NC_001959.2), GI.3 (Norwalk-like virus NLV/Honolulu/219/1992/US, NCBI access number: AF414403) and GII.2 (Norovirus GII.2 strain Env/CHN/2016/Gll.P16-GII.2/BJSMQ, NCBI access number: NC_039476) of norovirus was used.

### <Example 6-1>

In FIG. 6a, the gene of VP1 derived from the protein of norovirus genotype GI.1 (Norovirus Hu/Gl.1/8Mclll/1973/USA, NCBI access number: NC_001959.2) was used. VP1, which is an antigen of norovirus, consists of about 500 amino acids, and the molecular weight of the protein is about 55 to 60 kDa. Based on the protein sequence, a gene sequence synthesized after codon optimization in *E. coli* was prepared (SEQ ID NO. 12) (Request for gene synthesis to Bionics Co., Ltd.).

The gene of the above sequence was prepared based on pET-9a-EE domain (MSAVKAA)-SE domain (WT)-Nov GII.17 VP1, and pET-9a- EE domain (MSAVKAA)-m3c-Nov GII.17 VP1 vector, which is a recombinant complex protein expression vector prepared in Example 1-1 above, by using the pET-9a (Novagene, 69431-3CN) (SEQ ID NO: 1) expression vector. T7 RNA polymerase is expressed by IPTG, and through this, in the pET vector in which the Lac operon and T7 promoter present in the DE3 genome operate, at the existing N-terminus inserted into the multiple cloning site (MCS), the C-direction of the RID sequence for increasing solubility and expression rate, the 6xHIS, and the junction sequence for enhancing TEV cleavage efficiency was cut by using the Kpnl and BamHI restriction enzymes, and was subcloned and reinserted. Among the cleavage enzyme sites that are present within the MCS, the RID, cleavage enzyme site and linker site for increasing the cleavage efficiency of the TEV enzyme were inserted between the Ndel and Kpnl sequences, and DNA sequences designed to have a protein in the form of a fusion of the norovirus VP1 antigen protein were inserted between the Kpnl and BamHI cleavage enzymes, respectively, and each gene was linked to the pET vector and the inserted gene sequence through T4 DNA ligase. The bacterial clones generated by the DNA ligation reaction thereof were confirmed to have inserted the sequences of codon-optimized nucleotides through DNA sequence analysis.

A recombinant protein was prepared by fusing an RID EE domain peptide (MSAVKAA (SEQ ID NO: 2)) that has the effect of increasing the expression level and increasing the solubility at the N-terminus of the protein and an RID SE domain peptide (SEQ ID NO: 3) or a mutant RID peptide (SEQ ID NO: 7) that is more than 50% similar to SEQ ID NO: 3 and includes mutations of K23A, K30A, and K31A, and the solubility expression pattern was verified.

In addition, a peptide with an amino acid sequence of (GaSb)ₙ (a≥1, b≥1, n≥1) was used as a linker of an optimized sequence by applying the sequence of the TEV cleavage site, which is ENLYFQG/S, and this was included between the TEV cleavage sequence and the target protein (norovirus antigen protein) sequence.

The sequences of the recombinant complex expression vectors confirmed in Example 4-1 using the expression vector pET-9a (Novagene, 69431-3CN) (SEQ ID NO: 1) including the target protein of the above sequence are two types as follows:
EE domain (MSAVKAA) - SE domain - GT - 6xHIS - TEV - GS - NoV GI.1 VP1 [(SEQ ID NO: 2) - (SEQ ID NO: 3) - GT - 6xHIS - TEV - GS - (SEQ ID NO: 12]

:MSAVKAA-
AAVQAAEVKVDGSEPKLSKNELKRRLKAEKKVAEKEAKQKELSEKQLSQATAAATNHTTDNGV
GPEEESV-GT-HHHHHH-ENLYFQ-GS-
MMMASKDATSSVDGASGAGQLVPEVNASDPLAMDPVAGSSTAVATAGQVNPIDPWIINNF
VQAPQGEFTISPNNTPGDVLFDLSLGPHLNPFLLHLSQMYNGWVGNMRVRIMLAGNAFTAG
KIIVSCIPPGFGSHNLTIAQATLFPHVIADVRTLDPIEVPLEDVRNVLFHNNDRNQQTMRLVCM
LYTPLRTGGGTGDSFVVAGRVMTCPSPDFNFLFLVPPTVEQKTRPFTLPNLPLSSLSNSRAPLPIS
SMGISPDNVQSVQFQNGRCTLDGRLVGTTPVSLSHVAKIRGTSNGTVINLTELDGTPFHPFEGP
APIGFPDLGGCDWHINMTQFGHSSQTQYDVDTTPDTFVPHLGSIQANGIGSGNYVGVLSWIS
PPSHPSGSQVDLWKIPNYGSSITEATHLAPSVYPPGFGEVLVFFMSKMPGPGAYNLPCLLPQEY
ISHLASEQAPTVGEAALLHYVDPDTGRNLGEFKAYPDGFLTCVPNGASSGPQQLPINGVFVFVS
WVSRFYQLKPVGTASSARGRLGLRR

The prepared complex above was named as "RID-NoV GI.1 VP1."

MSAVKAA-m3c-GT-6xHIS-TEV-GS-NoV GI.1 VP1 [(SEQ ID NO. 2)-(SEQ ID NO. 7)-GT-6xHIS-TEV-GS-(SEQ ID NO. 12)]

:MSAVKAA-
AAVQAAEVKVDGSEPKLSKNELARRLKAEAAVAEKEAKQKELSEKQLSQATAAATNHTTDNGV
GPEEESV
-GT-HHHHHH-ENLYFQ-GS-
MMMASKDATSSVDGASGAGQLVPEVNASDPLAMDPVAGSSTAVATAGQVNPIDPWIINNF
VQAPQGEFTISPNNTPGDVLFDLSLGPHLNPFLLHLSQMYNGWVGNMRVRIMLAGNAFTAG
KIIVSCIPPGFGSHNLTIAQATLFPHVIADVRTLDPIEVPLEDVRNVLFHNNDRNQQTMRLVCM
LYTPLRTGGGTGDSFVVAGRVMTCPSPDFNFLFLVPPTVEQKTRPFTLPNLPLSSLSNSRAPLPIS
SMGISPDNVQSVQFQNGRCTLDGRLVGTTPVSLSHVAKIRGTSNGTVINLTELDGTPFHPFEGP
APIGFPDLGGCDWHINMTQFGHSSQTQYDVDTTPDTFVPHLGSIQANGIGSGNYVGVLSWIS
PPSHPSGSQVDLWKIPNYGSSITEATHLAPSVYPPGFGEVLVFFMSKMPGPGAYNLPCLLPQEY
ISHLASEQAPTVGEAALLHYVDPDTGRNLGEFKAYPDGFLTCVPNGASSGPQQLPINGVFVFVS
WVSRFYQLKPVGTASSARGRLGLRR

The prepared complex above was named as "RID(EE domain+m3c)-NoV GI.1 VP1."

### <Example 6-2>

The two expression vectors of Example 5-1 above were transformed into HMS174 competent cells (Novagen (RecA mutation in a K-12 strain, Cat: 69453-3)). All transformed *E. coli* were cultured in 3 mL of LB medium including 50 µg/mL kanamycin at 37°C for 5 to 7 hours at 250 rpm, and then, 1 mL was transferred to 10 mL, diluted 10-fold and further cultured. After about 2 to 4 hours, when O.D600 reached 0.5 to 0.7, 0.4 mM IPTG (Biosesang, Cat#I1006, Lot#LB0C0340, cas#367-93-1) was added and cultured at 16°C for 17 to 19 hours (maximum 21 hours). The culture solution was centrifuged to obtain only the precipitated *E. coli* and stored in an ultra-low temperature freezer at -80°C. 0.3 mL of lysis buffer (50 mM Tris-HCl (pH 7.5), 300 mM NaCl, 5% glycerol, 0.1% β-Mercaptoethanol and 10 mM imidazole) was added to the *E. coli* harvest corresponding to 3 mL of culture medium, and it was sonicated. The total lysate (T) was separated into the precipitant (P) and the soluble lysate (S) by centrifugation, and as a result of analysis by SDS-PAGE, it was confirmed that the norovirus GI.1 VP1 protein with the mutant RID added to the N-terminus exhibited a highly efficient soluble expression, as shown in FIG. 5a.

### <Example 6-3>

In FIG. 6b, the gene of VP1 derived from the protein of norovirus genotype GI.3 (Norwalk-like virus NLV/Honolulu/219/1992/US, NCBI access number: AF414403) was used. VP1, which is an antigen of norovirus, consists of about 500 amino acids, and the molecular weight of the protein is about 55 to 60 kDa. Based on the protein sequence, a gene sequence synthesized after codon optimization in *E. coli* was prepared (SEQ ID NO: 13) (Request for gene synthesis to Bionics Co., Ltd.).

The gene of the above sequence was prepared based on pET-9a-EE domain (MSAVKAA)-SE domain (WT)-Nov GII.17 VP1, pET-9a- EE domain (MSAVKAA)-m3c-Nov GII.17 VP1 vector, which is a recombinant complex protein expression vector prepared in Example 1-1 above, by using the pET-9a (Novagene, 69431-3CN) (SEQ ID NO: 1) expression vector. T7 RNA polymerase is expressed by IPTG, and through this, the RID sequence for increasing solubility and expression rate was cut off by using Kpnl and BamHI cleavage enzyme in the C-direction of the junction site sequence for improving 6xHIS and TEV cleavage efficiency, and was subcloned and reinserted at the existing N-terminus which was inserted into the multiple cloning site (MCS) of the pET vector in which the Lac operon and T7 promoter present in the DE3 genome operate. Among the cleavage enzyme sites that are present within the MCS, the RID, cleavage enzyme site and linker site of this study for increasing the cleavage efficiency of the TEV enzyme were inserted between the Ndel and Kpnl sequences, and DNA sequences designed to have a protein in the form of a fusion of the norovirus VP1 antigen protein were inserted between the Kpnl and BamHI cleavage enzymes, respectively, and each gene was linked to the pET vector and the inserted gene sequence through T4 DNA ligase. The bacterial clones generated by the DNA ligation reaction thereof were confirmed to have inserted the sequences of codon-optimized nucleotides through DNA sequence analysis.

A recombinant protein was prepared by fusion of MSAVKAA (SEQ ID NO: 2), which has the effect of increasing expression level and solubility at the N-terminus of the protein, with the RID peptide (SEQ ID NO: 3) or a mutant SE domain peptide (SEQ ID NO: 7) that is more than 50% similar to SEQ ID NO: 3 and includes mutations of K23A, K30A and K31A, and the solubility expression pattern was verified.

In addition, a peptide having an amino acid sequence of (GaSb)ₙ (a≥1, b≥1, n≥1) was used as a linker of an optimized sequence by applying the sequence of the TEV cleavage site, ENLYFQG/S, and this was included between the TEV cleavage sequence and the target protein sequence (norovirus antigen protein).

The sequences of the recombinant complex expression vector confirmed in Example 5-3 by using the expression vector pET-9a (Novagene, 69431-3CN) (SEQ ID NO: 3) including the target protein of the above sequence are two types as follows:
EE domain (MSAVKAA)-SE domain-GT-6xHIS-TEV-GS-NoV GI.3 VP1 [(SEQ ID NO: 2)-(SEQ ID NO: 3)-GT-6xHIS-TEV-GS-(SEQ ID NO: 13)]

:MSAVKAA-
AAVQAAEVKVDGSEPKLSKNELKRRLKAEKKVAEKEAKQKELSEKQLSQATAAATNHTTDNGV
GPEEESV-GT-HHHHHH-ENLYFQ-GS-
MMMASKDAPPNMDGTSGAGQLVPEANTAEPISMEPVAGAATAAATAGQVNMIDPWIMN
NYVQAPQGEFTISPNNTPGDILFDLQLGPHLNPFLSHLAQMYNGWVGNMKVKVLLAGNAFT
AGKIIISCIPPGFAAQNISIAQATMFPHVIADVRVLEPIEVPLEDVRNVLFHNNDNTPTMRLVCM
LYTPLRASGSSSGTDPFVIAGRVLTCPSPDFSLLFLVPPNVEQKTKPFSVPNLPLNTLSNSRVPSLI
KSMMVSRDHGQMVQFQNGRVALDGQLQGTTPTSASQLCKIRGSVFHANGGNGYNPTELD
GSPYHAFESPAPIGFPDLGECDWHMEASPTTQFDTGDVIKQINVKQESAFAPHLGTIQADGLS
DVSVNTNMIAKLGWVSPVSDGHRGNVDPWVIPRYGSTLTEAAQLAPPIYPPGFGEAIVFFMS
DFPIAHGTNGLSVPCTIPQEFVTHFVNEQAPTRGEAALLHYLDPDTHRNLGEFKLYPEGFMTCV
PNSSGTGPQTLPINGVFVFVSWVSRFYQLKPVGTAGPARRLGIRRS

The prepared complex above was named as "RID-NoV GI.3 VP1."

MSAVKAA-m3c-GT-6xHIS-TEV-GS-NoV GI.3 VP1 [(SEQ ID NO. 2)-(SEQ ID NO. 7)- GT-6xHIS-TEV-GS-(SEQ ID NO. 13)]

:MSAVKAA-
AAVQAAEVKVDGSEPKLSKNELARRLKAEAAVAEKEAKQKELSEKQLSQATAAATNHTTDNGV
GPEEESV
-GT-HHHHHH-ENLYFQ-GS-
MMMASKDAPPNMDGTSGAGQLVPEANTAEPISMEPVAGAATAAATAGQVNMIDPWIMN
NYVQAPQGEFTISPNNTPGDILFDLQLGPHLNPFLSHLAQMYNGWVGNMKVKVLLAGNAFT
AGKIIISCIPPGFAAQNISIAQATMFPHVIADVRVLEPIEVPLEDVRNVLFHNNDNTPTMRLVCM

LYTPLRASGSSSGTDPFVIAGRVLTCPSPDFSLLFLVPPNVEQKTKPFSVPNLPLNTLSNSRVPSLI
KSMMVSRDHGQMVQFQNGRVALDGQLQGTTPTSASQLCKIRGSVFHANGGNGYNPTELD
GSPYHAFESPAPIGFPDLGECDWHMEASPTTQFDTGDVIKQINVKQESAFAPHLGTIQADGLS
DVSVNTNMIAKLGWVSPVSDGHRGNVDPWVIPRYGSTLTEAAQLAPPIYPPGFGEAIVFFMS
DFPIAHGTNGLSVPCTIPQEFVTHFVNEQAPTRGEAALLHYLDPDTHRNLGEFKLYPEGFMTCV
PNSSGTGPQTLPINGVFVFVSWVSRFYQLKPVGTAGPARRLGIRRS

The prepared complex above was named as "RID(EE domain+m3c)-NoV GI.3 VP1."

### <Example 6-4>

The two expression vectors of Example 6-3 above were transformed into HMS174 competent cells (Novagen (RecA mutation in a K-12 strain, Cat: 69453-3)). All transformed *E. coli* were cultured in 3 mL of LB medium including 50 µg/mL kanamycin at 37°C for 5 to 7 hours at 250 rpm, and then, 1 mL was transferred to 10 mL, diluted 10-foldr and further cultured. After about 2 to 4 hours, when O.D600 reached 0.5 to 0.7, 0.4 mM IPTG (Biosesang, Cat#I1006, Lot#LB0C0340, cas#367-93-1) was added, and it was cultured at 16°C for 17 to 19 hours (maximum 21 hours). The culture solution was centrifuged to obtain only the precipitated *E. coli* and stored in an ultra-low temperature freezer at -80°C. 0.3 mL of a lysis buffer solution (50 mM Tris-HCl (pH 7.5), 300 mM NaCl, 5% glycerol, 0.1% β-Mercaptoethanol and 10 mM imidazole) was added to the *E. coli* harvest corresponding to 3 mL of culture medium, and it was sonicated. Afterwards, the total lysate (T) was separated into the precipitant (P) and soluble lysate (S), and as a result of analysis by SDS-PAGE, it was confirmed that the norovirus GI.3 VP1 protein with the mutant RID added to the N-terminus showed a high-efficiency soluble expression, as shown in FIG. 5b.

### <Example 6-5>

In FIG. 6c, the VP1 gene derived from the protein of Norovirus genotype GII.2 (Norovirus GII.2 strain Env/CHN/2016/GII.P16-GII.2/BJSMQ, NCBI access number: NC_039476) was used. VP1, which is an antigen of Norovirus, consists of about 500 amino acids, and the molecular weight of the protein is about 55 to 60 kDa. Based on the protein sequence above, a gene sequence synthesized after codon optimization in E. coli was prepared (SEQ ID NO. 11). (Request for gene synthesis to Bionics Co., Ltd.).

The gene of the above sequence was prepared based on the pET-9a-MSAVKAA-RID-Nov GII.17 VP1, pET-9a-MSAVKAA-m3c-Nov GII.17 vector, which is a recombinant complex protein expression vector prepared in Example 1, by using the pET-9a (Novagene, 69431-3CN) (SEQ ID NO: 1) expression vector. T7 RNA polymerase is expressed by IPTG, and through this, the RID sequence for increasing solubility and expression rate was cut off by using Kpnl and BamHI cleavage enzyme in the C-direction of the junction site sequence for improving 6xHIS and TEV cleavage efficiency, and was subcloned and reinserted at the existing N-terminus which was inserted into the multiple cloning site (MCS) of the pET vector in which the Lac operon and T7 promoter present in the DE3 genome operate. Among the cleavage enzyme sites that are present within the MCS, the RID, cleavage enzyme site and linker site of this study for increasing the cleavage efficiency of the TEV enzyme were inserted between the Ndel and Kpnl sequences, and DNA sequences designed to have a protein in the form of a fusion of the norovirus VP1 antigen protein were inserted between the Kpnl and BamHI cleavage enzymes, respectively, and each gene was linked to the pET vector and the inserted gene sequence through T4 DNA ligase. The bacterial clones generated by the DNA ligation reaction thereof were confirmed to have inserted the sequences of codon-optimized nucleotides through DNA sequence analysis.

A recombinant protein was prepared by fusing an EE domain peptide (MSAVKAA (SEQ ID NO: 2)) that has the effect of increasing the expression level and increasing the solubility at the N-terminus of the target protein (norovirus antigen protein) and an SE domain peptide (SEQ ID NO: 3) or a mutant SE domain peptide (SEQ ID NO: 7) that is more than 50% similar to SEQ ID NO: 3 and includes mutations of K23A, K30A and K31A, and the solubility expression pattern was verified.

In addition, a peptide with an amino acid sequence of (GaSb)ₙ (a≥1, b≥1, n≥1) was used as a linker of an optimized sequence by applying the sequence of the TEV cleavage site, ENLYFQG/S, and this was included between the TEV cleavage sequence and the target protein sequence (norovirus antigen protein).

The sequences of the recombinant complex expression vectors confirmed in Example 6-5 using the expression vector pET-9a (Novagene, 69431-3CN) (SEQ ID NO: 1) including the target protein of the above sequence are two types as follows:
MSAVKAA-RID-GT-6xHIS-TEV-GS-NoV GII.2 VP1 [(SEQ ID NO: 2)-(SEQ ID NO: 3)-GT-6xHIS-TEV-GS-(SEQ ID NO: 11)]

:MSAVKAA-
AAVQAAEVKVDGSEPKLSKNELKRRLKAEKKVAEKEAKQKELSEKQLSQATAAATNHTTDNGV
GPEEESV-GT-HHHHHH-ENLYFQ-GS-
MKMASNDAAPSTDGAAGLVPESNNEVMALEPVAGAALAAPVTGQTNIIDPWIRANFVQAP
NGEFTVSPRNAPGEVLLNLELGPELNPYLAHLARMYNGYAGGMEVQVMLAGNAFTAGKLVF
AAVPPHFPVENLSPQQITMFPHVIIDVRTLEPVLLPLPDVRNNFFHYNQKDDPKMRIVAMLYT
PLRSNGSGDDVFTVSCRVLTRPSPDFDFTYLVPPTVESKTKPFTLPILTLGELSNSRFPVSIDQMY
TSPNEIISVQCQNGRCTLDGELQGTTQLQVSGICAFKGEVTAHLHDNDHLYNVTITNLNGSPFD
PSEDIPAPLGVPDFQGRVFGIISQRDKHNSPGHNEPANRGHDAVVPTYTAQYTPKLGQIQIGT
WQTDDLTVNQPVKFTPVGLNDTEHFNQWVVPRYAGALNLNTNLAPSVAPVFPGERLLFFRSY
IPLKGGYGNPAIDCLLPQEWVQHFYQEAAPSMSEVALVRYINPDTGRALFEAKLHRAGFMTVS
SNTSAPVVVPANGYFRFDSWVNQFYSLAPMGTGNGRRRVQ

The prepared complex above was named as "RID-NoV GII.2 VP1."

MSAVKAA-m3c-GT-6xHIS-TEV-GS-NoV GII.2 VP1 [(SEQ ID NO. 2)-(SEQ ID NO. 7)- GT-6xHIS-TEV-GS-(SEQ ID NO. 11)]

:MSAVKAA-
AAVQAAEVKVDGSEPKLSKNELARRLKAEAAVAEKEAKQKELSEKQLSQATAAATNHTTDNGV
GPEEESV
-GT-HHHHHH-ENLYFQ-GS-
MKMASNDAAPSTDGAAGLVPESNNEVMALEPVAGAALAAPVTGQTNIIDPWIRANFVQAP
NGEFTVSPRNAPGEVLLNLELGPELNPYLAHLARMYNGYAGGMEVQVMLAGNAFTAGKLVF
AAVPPHFPVENLSPQQITMFPHVIIDVRTLEPVLLPLPDVRNNFFHYNQKDDPKMRIVAMLYT
PLRSNGSGDDVFTVSCRVLTRPSPDFDFTYLVPPTVESKTKPFTLPILTLGELSNSRFPVSIDQMY
TSPNEIISVQCQNGRCTLDGELQGTTQLQVSGICAFKGEVTAHLHDNDHLYNVTITNLNGSPFD
PSEDIPAPLGVPDFQGRVFGIISQRDKHNSPGHNEPANRGHDAVVPTYTAQYTPKLGQIQIGT
WQTDDLTVNQPVKFTPVGLNDTEHFNQWVVPRYAGALNLNTNLAPSVAPVFPGERLLFFRSY
IPLKGGYGNPAIDCLLPQEWVQHFYQEAAPSMSEVALVRYINPDTGRALFEAKLHRAGFMTVS
SNTSAPVVVPANGYFRFDSWVNQFYSLAPMGTGNGRRRVQ

The prepared complex above was named as "RID(EE domain+m3c)-NoV GII.2 VP1."

### <Example 6-6>

The two expression vectors of Example 6-5 above were transformed into HMS174 competent cells (Novagen (RecA mutation in a K-12 strain, Cat: 69453-3)). All transformed *E. coli* were cultured in 3 mL of LB medium including 50 µg/mL kanamycin at 37°C for 5 to 7 hours at 250 rpm, and then, 1 mL was transferred to 10 mL, diluted 10-fold and further cultured. After about 2 to 4 hours, when O.D600 reached 0.5 to 0.7, 0.4 mM IPTG (Biosesang, Cat#I1006, Lot#LB0C0340, cas#367-93-1) was added and cultured at 16°C for 17 to 19 hours (maximum 21 hours). The culture solution was centrifuged to obtain only the precipitated *E. coli* and stored in an ultra-low temperature freezer at -80°C. 0.3 mL of a lysis buffer solution (50 mM Tris-HCl (pH 7.5), 300 mM NaCl, 5% glycerol, 0.1% β-Mercaptoethanol and 10 mM imidazole) was added to the *E. coli* harvest corresponding to 3 mL of culture medium, and it was sonicated. Afterwards, the total lysate (T) was separated into the precipitant (P) and the soluble lysate (S) by centrifugation, and as a result of analysis by SDS-PAGE, it was confirmed that the norovirus GII.2 VP1 protein (fusion protein) with the mutant RID added to the N-terminus showed a high-efficiency soluble expression, as shown in FIG. 5c.

### <Example 6-7>

The VP1 of norovirus GI.1, GI.3 and GII.2 obtained in Example 6 above was purified in the same manner as in the previous example, and the purified norovirus VP1 protein was exchanged in the Assembly buffer including a 10% glycerol ratio for overnight (12 to 16 hours) and stored at 4°C. In order to determine the overall diameter distribution of the assembled virus-like particles (VLPs), it was analyzed through dynamic light scattering (DLS). The analysis of the complex was measured by placing 1 mL of the sample in a cuvette at a temperature of 16°C using DLS (Particular Systems, Zetasizer Nano Family). The dynamic light scattering analysis of the cell was confirmed by using the total intensity and mass of each area in the image.

In addition, the appearance of the virus-like particles (VLPs) was observed by an electron microscope. The purified norovirus VLPs were first placed on a copper grid for 1 minute, then stained with 2% uranyl acetate for 1 minute, dried at room temperature for 10 minutes, and then photographed by using a transmission electron microscope (TEM; 120 kV; Talos L120C, FEI, Czech). As shown in FIGS. 6d to 6d, the purified and assembled norovirus VP1 protein had a particle size distribution of 30 to 40 nm in diameter in the hydrodynamic radius of DLS, and it was confirmed that it formed VLPs. The diameter of the VLPs confirmed in TEM was between 30 and 40 nm, which is the diameter of the norovirus particle, and it was confirmed that it formed highly homogeneous spherical VLPs.

When the mutant RID according to the present invention and the norovirus antigen protein were fused, it was confirmed that it was essential for expressing the target protein with much higher efficiency in *E. coli,* inducing proper folding and improving VLP assembly ability.

Although the exemplary embodiments of the present invention have been described above, those skilled in the art will understand that the present invention can be implemented in other specific forms without changing the technical idea or essential features thereof. Therefore, it should be understood that the exemplary embodiments described above are exemplary in all respects and not restrictive.

### [Sequence List Free Text]

**SEQ ID NO: 1**
   **Vector Map (pET-9a)** 
ttctcatgtttgacagcttatcatcgataagctttaatgcggtagtttatcacagttaaattgctaacgcagtc
aggcaccgtgtatgaaatctaacaatgcgctcatcgtcatcctcggcaccgtcaccctggatgctgtaggcataggcttg
gttatgccggtactgccgggcctcttgcgggatatcgtccattccgacagcatcgccagtcactatggcgtgctgctagc
gctatatgcgttgatgcaatttctatgcgcacccgttctcggagcactgtccgaccgctttggccgccgcccagtcctgct
cgcttcgctacttggagccactatcgactacgcgatcatggcgaccacacccgtcctgtggata
tccggatatagttcctcctttcagcaaaaaacccctcaagacccgtttagaggccccaaggggttatgctagttattgctc
agcggtggcagcagccaactcagcttcctttcgggctttgttagcagccggatccgcgacccatttgctgtccaccagtc
atgctagccatatgtatatctccttcttaaagttaaacaaaattatttctagagggaaaccgttgtggtctccctatagtg
 
agtcgtattaatttcgcgggatcgagatctcgatcctctacgccggacgcatcgtggccggcatcaccggcgccacagg
tgcggttgctggcgcctatatcgccgacatcaccgatggggaagatcgggctcgccacttcggg
ctcatgagcgcttgtttcggcgtgggtatggtggcaggccccgtggccgggggactgttgggcgccatctccttgcatgc
accattccttgcggcggcggtgctcaacggcctcaacctactactgggctgcttcctaatgcaggagtcgcataaggga
gagcgtcgaccgatgcccttgagagccttcaacccagtcagctccttccggtgggcgcggggcatgactatcgtcgccg
cacttatgactgtcttctttatcatgcaactcgtaggacaggtgccggcagcgctctgggtcattttcggcgaggaccgct
ttcgctggagcgcgacgatgatcggcctgtcgcttgcggtattcggaatcttgcacgccc
tcgctcaagccttcgtcactggtcccgccaccaaacgtttcggcgagaagcaggccattatcgccggcatggcggccga
cgcgctgggctacgtcttgctggcgttcgcgacgcgaggctggatggccttccccattatgattcttctcgcttccggcgg
catcgggatgcccgcgttgcaggccatgctgtccaggcaggtagatgacgaccatcagggacagcttcaaggatcgct
cgcggctcttaccagcctaacttcgatcactggaccgctgatcgtcacggcgatttatgccgcctcggcgagcacatgga
acgggttggcatggattgtaggcgccgccctataccttgtctgcctccccgcgttgcgtcgcggtgc
atggagccgggccacctcgacctgaatggaagccggcggcacctcgctaacggattcaccactccaagaattggagcc
aatcaattcttgcggagaactgtgaatgcgcaaaccaacccttggcagaacatatccatcgcgtccgccatctccagca
gccgcacgcggcgcatctcgggcagcgttgggtcctggccacgggtgcgcatgatcgtgctcctgtcgttgaggacccg
gctaggctggcggggttgccttactggttagcagaatgaatcaccgatacgcgagcgaacgtgaagcgactgctgctgc
aaaacgtctgcgacctgagcaacaacatgaatggtcttcggtttccgtgtttcgtaaagtctgg
aaacgcggaagtcagcgccctgcaccattatgttccggatctgcatcgcaggatgctgctggctaccctgtggaacacc
tacatctgtattaacgaagcgctggcattgaccctgagtgatttttctctggtcccgccgcatccataccgccagttgttta
ccctcacaacgttccagtaaccgggcatgttcatcatcagtaacccgtatcgtgagcatcctctctcgtttcatcggtatc
attacccccatgaacagaaatcccccttacacggaggcatcagtgaccaaacaggaaaaaaccgcccttaacatggcc
cgctttatcagaagccagacattaacgcttctggagaaactcaacgagctggacgcggatgaaca
ggcagacatctgtgaatcgcttcacgaccacgctgatgagctttaccgcagctgcctcgcgcgtttcggtgatgacggtg
aaaacctctgacacatgcagctcccggagacggtcacagcttgtctgtaagcggatgccgggagcagacaagcccgtc
agggcgcgtcagcgggtgttggcgggtgtcggggcgcagccatgacccagtcacgtagcgatagcggagtgtatactg
gcttaactatgcggcatcagagcagattgtactgagagtgcaccatatatgcggtgtgaaataccgcacagatgcgtaa
ggagaaaataccgcatcaggcgctcttccgcttcctcgctcactgactcgctgcgctcggtcgt
tcggctgcggcgagcggtatcagctcactcaaaggcggtaatacggttatccacagaatcaggggataacgcaggaa
agaacatgtgagcaaaaggccagcaaaaggccaggaaccgtaaaaaggccgcgttgctggcgtttttccataggctcc
gcccccctgacgagcatcacaaaaatcgacgctcaagtcagaggtggcgaaacccgacaggactataaagataccag
gcgtttccccctggaagctccctcgtgcgctctcctgttccgaccctgccgcttaccggatacctgtccgcctttctcccttc
gggaagcgtggcgctttctcatagctcacgctgtaggtatctcagttcggtgtaggtcgttcgctccaag
ctgggctgtgtgcacgaaccccccgttcagcccgaccgctgcgccttatccggtaactatcgtcttgagtccaacccggt
aagacacgacttatcgccactggcagcagccactggtaacaggattagcagagcgaggtatgtaggcggtgctacag
agttcttgaagtggtggcctaactacggctacactagaaggacagtatttggtatctgcgctctgctgaagccagttacc
ttcggaaaaagagttggtagctcttgatccggcaaacaaaccaccgctggtagcggtggtttttttgtttgcaagcagca
gattacgcgcagaaaaaaaggatctcaagaagatcctttgatcttttctacggggtctgacg
ctcagtggaacgaaaactcacgttaagggattttggtcatgaacaataaaactgtctgcttacataaacagtaatacaa
ggggtgttatgagccatattcaacgggaaacgtcttgctcgaggccgcgattaaattccaacatggatgctgatttatat
gggtataaatgggctcgcgataatgtcgggcaatcaggtgcgacaatctatcgattgtatgggaagcccgatgcgcca
 
gagttgtttctgaaacatggcaaaggtagcgttgccaatgatgttacagatgagatggtcagactaaactggctgacgg
aatttatgcctcttccgaccatcaagcattttatccgtactcctgatgatgcatggttactcaccactg
cgatccccgggaaaacagcattccaggtattagaagaatatcctgattcaggtgaaaatattgttgatgcgctggcagt
gttcctgcgccggttgcattcgattcctgtttgtaattgtccttttaacagcgatcgcgtatttcgtctcgctcaggcgcaat
cacgaatgaataacggtttggttgatgcgagtgattttgatgacgagcgtaatggctggcctgttgaacaagtctggaa
agaaatgcataagcttttgccattctcaccggattcagtcgtcactcatggtgatttctcacttgataaccttatttttgac
gaggggaaattaataggttgtattgatgttggacgagtcggaatcgcagaccgata
ccaggatcttgccatcctatggaactgcctcggtgagttttctccttcattacagaaacggctttttcaaaaatatggtatt
gataatcctgatatgaataaattgcagtttcatttgatgctcgatgagtttttctaagaa
**SEQ ID NO: 2**
   **RID EE domain**
   MSAVKAA
**SEQ ID NO: 3**
   **RID SE domain** 
AAVQAAEVKVDGSEPKLSKNELKRRLKAEKKVAEKEAKQKELSEKQLSQATAAATNH
TTDNGVGPEEESV
**SEQ ID NO: 4**
   **RID SE domain m9** 
AAVQAAEVKVDGSEPKLSANELAARLAAEAAVAEAEAAQAELSEKQLSQATAAATN
HTTDNGVGPEEESV
**SEQ ID NO: 5**
   RID SE domain m3a 
AAVQAAEVKVDGSEPKLSANELKRRLKAEKKVAEKEAAQAELSEKQLSQATAAATNH
TTDNGVGPEEESV
SEQ ID NO: 6
   **RID SE domain m3b** 
AAVQAAEVKVDGSEPKLSANELAARLKAEKKVAEKEAKQKELSEKQLSQATAAATNH
TTDNGVGPEEESV
**SEQ ID NO: 7**
   **RID SE domain m3c** 
AAVQAAEVKVDGSEPKLSKNELARRLKAEAAVAEKEAKQKELSEKQLSQATAAATNH
TTDNGVGPEEESV
**SEQ ID NO: 8**
   **RID SE domain m4** 
AAVQAAEVKVDGSEPKLSKNELAARLKAEAAVAEKEAKQKELSEKQLSQATAAATNH
TTDNGVGPEEESV
**SEQ ID NO: 9**
   **Norovirus Genotype GII.17 (Norovirus Hu/GII.17/HKG/2014/CUHK-NS-405; Gene ID: KP902566.1)** 
MKMASNDAAPSNDGAAGLVPEGNNETLPLEPVAGAAIAAPVTGQNNIIDPWIRTN
FVQAPNGEFTVSPRNSPGEILLNLELGPDLNPYLAHLSRMYNGYAGGVEVQVLLAGNAFTAGK
ILFAAVPPNFPVEFLSPAQITMLPHLIVDVRTLEPIMIPLPDVRNTFFHYSNQPNSRMRLVAMLY
TPLRSNGSGDDVFTVSCRVLTRPTPDFEFTYLVPPSVESKTKPFSLPILTLSELTNSRFPVPIDSLFT
AQNNVLQVQCQNGRCTLDGELQGTTQLLPSGICAFRGRVTAQINQRDRWHMQLQNLNGTT
YDPTDDVPAPLGTPDFKGVVFGMVSQRNVGNDAPGSTRAQQAWVSTYSPQFVPKLGSVNLR
ISDNDDFQFQPTKFTPVGVNDDDDGHPFRQWELPNYSGELTLNMNLAPPVAPNFPGEQLLFF
RSFVPCSGGYNQGIIDCLIPQEWIQHFYQESAPSQSDVALIRYVNPDTGRTLFEAKLHRSGYITV
AHSGDYPLVVPANGHFRFDSWVNQFYSLAPMGTGNGRRRAQ
**SEQ ID NO: 10**
   **Norovirus Genotype GII.3 (Norovirus Hu/G11.3/CHDC2005/1975/US; Gene ID: HM072045.1)** 
MKMASNDAAPSNDGAAGLVPEINNEAMALEPVAGAAIAAPLTGQQNIIDPWIMN
NFVQAPGGEFTVSPRNSPGEVLLNLELGPEINPYLAHLARMYNGYAGGFEVQVVLAGNAFTA
GKVIFAAIPPNFPIDNLSAAQITMCPHVIVDVRQLEPINLPMPDVRNNFFHYNQGSDSRLRLIA
MLYTPLRANNSGDDVFTVSCRVLTRPSPDFSFNFLVPPTVESKTKPFTLPILTISEMSNSRFPVPI
DSLHTSPTENIVVQCQNGRVTLDGELMGTTQLLPSQICAFRGTLTRSTSRAGDQADTATPRLF
NYYWHIQLDNLNGTPYDPAEDIPAPLGTPDFRGKVFGVASQRNPDSTTRAHEAKVDTTSGRF
APKLGSLEISTESSDFDSNQPTRFTPVGIGVDNEADFQQWSLPDYSGQFTHNMNLAPAVAPNF
PGEQLLFFRSQLPSSGGRSNGILDCLVPQEWVQHFYQESAPAQTQVALVRYVNPDTGRVLFEA
KLHKLGFMTIAKNGDSPITVPPNGYFRFESWVNPFYTLAPMGTGNGRRRIQ
**SEQ ID NO: 11**
   **Norovirus Genotype GII.2 (Norovirus GII.2 strain Env/CHN/2016/GII.P16-GII.2/BJSMQ; Gene ID: NC_039476)** 
MKMASNDAAPSTDGAAGLVPESNNEVMALEPVAGAALAAPVTGQTNIIDPWIRANFVQAP
NGEFTVSPRNAPGEVLLNLELGPELNPYLAHLARMYNGYAGGMEVQVMLAGNAFTAGKLVF
AAVPPHFPVENLSPQQITMFPHVIIDVRTLEPVLLPLPDVRNNFFHYNQKDDPKMRIVAMLYT
PLRSNGSGDDVFTVSCRVLTRPSPDFDFTYLVPPTVESKTKPFTLPILTLGELSNSRFPVSIDQMY
TSPNEIISVQCQNGRCTLDGELQGTTQLQVSGICAFKGEVTAHLHDNDHLYNVTITNLNGSPFD
PSEDIPAPLGVPDFQGRVFGIISQRDKHNSPGHNEPANRGHDAVVPTYTAQYTPKLGQIQIGT
WQTDDLTVNQPVKFTPVGLNDTEHFNQWVVPRYAGALNLNTNLAPSVAPVFPGERLLFFRSY
 
IPLKGGYGNPAIDCLLPQEWVQHFYQEAAPSMSEVALVRYINPDTGRALFEAKLHRAGFMTVS
SNTSAPVVVPANGYFRFDSWVNQFYSLAPMGTGNGRRRVQ
**SEQ ID NO: 12**
   **Norovirus Genotype GI.1(Norovirus Hu/GI.1/8McIII3/1973/USA; Gene ID: KF429770.1, NC_001959.2)** 
MMMASKDATSSVDGASGAGQLVPEVNASDPLAMDPVAGSSTAVATAGQVNPIDPWIINNF
VQAPQGEFTISPNNTPGDVLFDLSLGPHLNPFLLHLSQMYNGWVGNMRVRIMLAGNAFTAG
KIIVSCIPPGFGSHNLTIAQATLFPHVIADVRTLDPIEVPLEDVRNVLFHNNDRNQQTMRLVCM
LYTPLRTGGGTGDSFVVAGRVMTCPSPDFNFLFLVPPTVEQKTRPFTLPNLPLSSLSNSRAPLPIS
SMGISPDNVQSVQFQNGRCTLDGRLVGTTPVSLSHVAKIRGTSNGTVINLTELDGTPFHPFEGP
APIGFPDLGGCDWHINMTQFGHSSQTQYDVDTTPDTFVPHLGSIQANGIGSGNYVGVLSWIS
PPSHPSGSQVDLWKIPNYGSSITEATHLAPSVYPPGFGEVLVFFMSKMPGPGAYNLPCLLPQEY
ISHLASEQAPTVGEAALLHYVDPDTGRNLGEFKAYPDGFLTCVPNGASSGPQQLPINGVFVFVS
WVSRFYQLKPVGTASSARGRLGLRR
**SEQ ID NO: 13**
   **Norovirus Genotype GI.3 (Norwalk-like virus NLV/Honolulu/219/1992/US; Gene ID: AF414403)**

MMMASKDAPPNMDGTSGAGQLVPEANTAEPISMEPVAGAATAAATAGQVNMIDPWIMN
NYVQAPQGEFTISPNNTPGDILFDLQLGPHLNPFLSHLAQMYNGWVGNMKVKVLLAGNAFT
AGKIIISCIPPGFAAQNISIAQATMFPHVIADVRVLEPIEVPLEDVRNVLFHNNDNTPTMRLVCM
LYTPLRASGSSSGTDPFVIAGRVLTCPSPDFSLLFLVPPNVEQKTKPFSVPNLPLNTLSNSRVPSLI
KSMMVSRDHGQMVQFQNGRVALDGQLQGTTPTSASQLCKIRGSVFHANGGNGYNPTELD
GSPYHAFESPAPIGFPDLGECDWHMEASPTTQFDTGDVIKQINVKQESAFAPHLGTIQADGLS
DVSVNTNMIAKLGWVSPVSDGHRGNVDPWVIPRYGSTLTEAAQLAPPIYPPGFGEAIVFFMS
DFPIAHGTNGLSVPCTIPQEFVTHFVNEQAPTRGEAALLHYLDPDTHRNLGEFKLYPEGFMTCV
PNSSGTGPQTLPINGVFVFVSWVSRFYQLKPVGTAGPARRLGIRRS

## Claims

1. A recombinant expression vector for producing a norovirus vaccine, comprising a polynucleotide encoding:
a norovirus antigen protein; and
a mutant protein in which 3 or 4 amino acid residues in the sequence of RNA interacting domain (RID) isolated from mammalian lysyl tRNA synthetase (mammalian LysRS) are substituted with other amino acid residues.

2. The recombinant expression vector of claim 1, wherein the RID comprises the amino acid sequences of SEQ ID NO: 2 and SEQ ID NO: 3.

3. The recombinant expression vector of claim 1, wherein the substituted amino acid residues comprise at least any one of the 19^{th}, 23^{rd}, 24^{th}, 30^{th}, 31^{st}, 38^{th} and 40^{th} amino acid residues in the amino acid sequence of SEQ ID NO: 3.

4. The recombinant expression vector of claim 1, wherein the mutant protein of the RID comprises any one of the amino acid sequences of SEQ ID NO: 5 to SEQ ID NO: 8.

5. The recombinant expression vector of claim 1, wherein the norovirus antigen protein comprises at least any one amino acid sequence selected from the group consisting of SEQ ID NOs: 9 to 13.

6. A host cell, which is transformed by the expression vector according to any one of claims 1 to 5.

7. The host cell of claim 6, wherein the host cell is *E. coli.*

8. A fusion protein for a norovirus vaccine, comprising:
a norovirus antigen protein; and
a mutant protein in which 3 or 4 amino acid residues in the sequence of RNA interacting domain (RID) isolated from mammalian lysyl tRNA synthetase (mammalian LysRS) are substituted with other amino acid residues.

9. The fusion protein of claim 8, wherein the RID comprises the amino acid sequences of SEQ ID NO: 2 and SEQ ID NO: 3, and
wherein the substituted amino acid residues comprise at least any one of the 19^{th}, 23^{rd}, 24^{th}, 30^{th}, 31^{st}, 38^{th} and 40^{th} amino acid residues in the amino acid sequence of SEQ ID NO: 3.

10. The fusion protein of claim 8, wherein the mutant protein of the RID comprises at least any one of the amino acid sequences of SEQ ID NO: 5 to SEQ ID NO: 8.

11. The fusion protein of claim 8, wherein the norovirus antigen protein comprises at least any one amino acid sequence selected from the group consisting of SEQ ID NOs: 9 to 13.

12. A virus-like particle, which is formed by self-assembly of an antigen protein cleaved from the fusion protein of claim 8.

13. A method for producing norovirus-like particles, comprising the steps of:
a) producing a recombinant expression vector for producing a vaccine, comprising a polynucleotide encoding: a norovirus antigen protein; and a mutant protein in which 3 or 4 amino acid residues in the sequence of RNA interacting domain (RID) isolated from mammalian lysyl tRNA synthetase (mammalian LysRS) are substituted with other amino acid residues;
b) producing a transformant by introducing the expression vector into a host cell; and
c) culturing the transformant to induce the expression of a recombinant fusion protein and then obtaining the recombinant fusion protein.

14. The method of claim 13, wherein the prepared fusion protein comprises a tobacco etch virus (TEV) protein between the norovirus antigen protein and the RID protein, and
wherein the method comprises the step of d) cleaving between the RID protein of the prepared fusion protein and the norovirus antigen protein, by using TEV cleavage enzyme, after step c).

15. The method of claim 14, further comprising the step of:
e) purifying the cleaved antigen protein, after step d).

16. The method of claim 15, further comprising a step in which the purified protein is self-assembled to form virus-like particles, after step e).

17. The method of claim 13, wherein the RID comprises the amino acid sequences of SEQ ID NO: 2 and SEQ ID NO: 3, and
wherein the substituted amino acid residues comprise at least any one of the 19^{th}, 23^{rd}, 24^{th}, 30^{th}, 31^{st}, 38^{th} and 40^{th} amino acid residues in the amino acid sequence of SEQ ID NO: 3.

18. The method of claim 13, wherein the mutant protein of the RID comprises any one of the amino acid sequences of SEQ ID NO: 5 to SEQ ID NO: 8.

19. The method of claim 13, wherein the norovirus antigen protein comprises at least any one amino acid sequence selected from the group consisting of SEQ ID NOs: 9 to 13.
